# EUROPEAN PATENT SPECIFICATION

(11) **EP 2 805 732 B1**
(45) Date of publication and mention of the grant of the patent: **28.09.2016**
(21) Application number: 13382184.3
(22) Date of filing: 20.05.2013
(51) Int. Cl.: A61K 47/48, A61K 49/00, A61K 49/14, A61K 51/08, A61P 25/28

(54) **Polymer-drug conjugates for the treatment of amyloidosis**
Polymerwirkstoffkonjugate zur Behandlung von Amyloidose
Conjugués médicament-polymère pour le traitement de l'amyloïdose

(43) Date of publication of application: 26.11.2014
(73) Proprietor: Fundación Comunidad Valenciana Centro de Investigación Principe Felipe, 46012 Valencia (ES)
(72) Inventor: Vicent Docón, María Jesús, 12540 Vila-real (Castellón) (ES); Conejos Sanchez, Inmaculada, 46117 Betera (Valencia) (ES)
(74) Representative: Carlos Hernando, Borja

(56) References cited:
- WO-A1-2013/060919
- WO-A2-2008/124735
- US-A1- 2010 233 779

## Description

### FIELD OF THE INVENTION

The present invention relates to novel chemical conjugates as well as pharmaceutical compositions containing them for use in therapy and diagnosis and, more particularly, but not exclusively, to novel conjugates of polymers having attached thereto a targeting moiety and one or more therapeutic agents for the treatment of amyloidosis. The present invention is referred to novel polymeric conjugates which is linked at least to a fibril disruptor agent and/or a blocking agent of aggregates in addition of an optional targeting moiety and/or a probe for diagnosis and therapy.

Specifically, the present invention relates to a polymer-drug conjugate, where the polymeric platform transports at least one bioactive agent, selected from the group of anthracyclines antibiotics such as tetracycline, rolitetracycline, minocycline and/or doxycycline and their derivatives, able to disaggregate or break the amyloid fibrils and/or block the aggregates. This conjugate could contain in its structure one or several targeting moieties, which will provide an effective targeting of the polymer-drug conjugate to the selected area for drug activity, increasing the therapeutic efficacy in the treatment of amyloidosis.

### BACKGROUND OF THE INVENTION

Amyloidosis is a group of diseases characterized by fibrillar protein deposition in the extracellular space named amyloid, of which Familial Amyloid Polyneuropathy (FAP) and Alzheimer's Disease (AD) are examples.

Familial Amyloidotic Polyneuropathy (FAP) is a neurodegenerative disorder characterised by systemic extracellular deposition of transthyretin (TTR) amyloid fibrils in several organs, mainly in the peripheral nervous system. This disease is characterised by an ascending sensorimotor polyneuropathy and progressive dysautonomia, becoming usually fatal 10 to 15 years after its onset. TTR, a homotetramer mainly synthesised in the liver and choroid plexus, is responsible for the transport of thyroxine and retinol. Over 100 point mutations have been identified in TTR and associated with FAP; this disease presents its largest focus in Portugal and thus, represents an important topic of research. The mechanism by which TTR deposits and becomes pathological is not fully understood but it is currently accepted that the protein undergoes a series of events that lead to its dissociation into monomers which aggregate culminating with the formation of amyloid fibrils. Although with variable results, orthotopic liver transplantation (OLT) is recognized as the only treatment modality available for FAP. Currently, there is no effective treatment for this disorder and the search for drugs capable of interfering with the amyloidogenic cascade has been quite intense.
Underlying therapeutic strategies is the need to identify and to characterise TTR species formed along the process and the identification of biomarkers to assess disease progression and follow up of treatments. Saraiva et al. previously contributed for the characterisation of the dynamics of TTR fibril formation, including the characterisation of the different TTR species generated along the process of fibril formation [1] which allowed the screening of different drugs in vitro, acting at different stages of the amyloidogenic [2, 3], and in vivo[4]. The drug selected in the present invention, doxycycline (Doxy), is the most advanced fibril disrupter agent under study, being the most effective compound at disaggregating TTR mature fibrils. Doxy was able to disrupt amyloid fibrils *in vitro,* in the treated animals and also achieved other improvements regarding amyloid markers [2, 4]. Combination of Doxy with Tauroursodeoxycholic Acid (TUDCA) is already in phase II of clinical trials. TUDCA is a biliary acid with antiapoptotic and antioxidant activity. In recent studies, the combination of doxycycline with TUDCA administered to mice with amyloid deposition was more effective than either parent drugs per separate. Significantly lowering of TTR deposition and associated tissue markers were observed. The observed synergistic effect of doxy/TUDCA in the range of human tolerable quantities, in the transgenic TTR mice models prompts their application in FAP, particularly in the early stages of disease. During this study, it was suggested that a possible mechanism for TTR extracellular aggregation is the influence of secreted metabolites generated by oxidative stress and apoptosis on TTR [5]. The neuroprotector effect of this class of compounds, in addition to its own anti-microbacterial properties, was described in several other disease models including cerebral ischemia, spinal cord injury, Parkinson's disease (PD), Huntington's disease (HD), Amyotrophic lateral sclerosis (ALS), Multiple Sclerosis (MS), and AD. Within this scope, Doxycycline in combination with rifampin were tested in clinical trials as antibiotic therapy in Alzheimer's disease [6] although there were no statistically significant decline/deterioration results in comparison with placebo [7, 8]. However, several publications evidence the beneficial properties of tetracyclines for AD treatment, such as their anti-amyloidogenic activity [2, 9-12]. It is noteworthy to remark that work carried out by Cardoso *et al.,* where doxycycline has demonstrate prevention of amyloid beta (Aβ) toxicity *in vitro* and *in vivo* [13].

In addition, it has been described that TTR binds A-beta protein, which plays the major role in the Alzheimer's Disease (AD). It can act like a "chaperone" by preventing formation of A-beta amyloid aggregates and thereby it may halt progression of AD [14]. This disorder, also an amyloidotic disease, affects 5% of the population over the age of 65 years and 20% over 80 years of age.

However, the advances in the design of novel drugs for amyloidosis treatment still reveal the need of more effective treatments by means of enhancing drug specificity and decreasing its systemic toxicity.

The present invention provides a response to this necessity with novel polymer-drug conjugates able to enhance the activity already found with doxycycline in the treatment of diseases involving amyloidosis, e.g. FAP, showing better specificity and reduced systemic toxicity.

Polymer Therapeutics, first polymeric nanomedicines [15, 16], are the basis for the development of the invention which is based on conjugation of the aforementioned compounds (directly or through a linker to a polymeric carrier) by covalent binding in order to obtain novel polymer-drug conjugates with therapeutic potential to treat these neuropathic disorders for the first time in the field. Conjugation procedures have demonstrated greater specificity and increased or retained activity of the parent molecules, importantly, polymer multivalence allows to achieve synergistic effect with combination therapy [17-19]). Conjugation can also provide improvements on drug stability and can reduce drug toxicity yielding a better therapeutic value.

Polymer Therapeutics are well-known as effective drug delivery systems with demonstrated clinical benefits since the 90's [20, 21]. In particular polymer-drug conjugates are considered new chemical entities (NCEs) capable to improve bioactive compound properties (changing its pharmacokinetics at whole body (EPR effect) and at cellular level (endocytosis) allowing even to overcome cellular mechanisms of resistance [15, 22]) and decreasing their inherent limitations (short half life, non-specific toxicity, low solubility, poor stability, potential immunogenicity,...). More than 16 polymer-drug conjugates have already achieved clinical development [23, 24], mainly as anticancer agents and currently, a second generation of conjugates focused on improved structures, combination therapy or new molecular targets are under development to move this platform technology further [20, 21, 23, 25, 26]. The conjugate paclitaxel-polyglutamate OPAXIO™ (PGA-PTX conjugate) already in Phase II trials is the most advanced [27, 28].

In the international patent application WO2007060524 it is also described certain compounds 1,4-diazepane-2,5-dione which are able to be conjugated to poly-glutamic acid through covalent binding such amide bond. They have been proposed for the treatment of PD, MS and stroke. The conjugate design described in this invention considers also the presence of a peptidic linker between the drug and the polymeric chain.

Furthermore, due to the molecular complexity of diseases (such as neurodegenerative disorders), combination therapy is becoming increasingly important for a better long-term prognosis and to decrease side effects. Therefore, one of the main targets of the present invention is the use of advanced nanopharmaceutics based on combination therapy looking at agent synergism. Whilst nanosized systems are well established for the delivery of a single therapeutic agent, only in very recent years has their use been extended to the delivery of multi-agent therapy. In fact, the only example in the clinics comes from a Canadian company, Celator Technologies Inc. who has developed a methodical approach for combination therapy within their liposomal technology [29-33]. This technology led to the development of different liposomal formulations that are now being assessed in Phase II clinical trials, namely CPX-1 and CPX-351. These early studies revealed the therapeutic potential of this application but raised new challenges that need to be addressed for a successful optimisation of the system towards clinical applications.

Following these concepts, novel specific nanoconjugates for the treatment of amyloidosis related polyneuropathies are shown in this invention, using as polymeric platform the poly-glutamic acid (PGA) and its derivates.

One of the major drawbacks that hinders peptides/proteins drugs application in therapies is their variable solubility, low bioavailability and limited stability, therefore one purpose of the present invention is to move a step further this therapy enhancing the activity already found with Doxy in FAP and AD with polymer-drug conjugates, increasing their specificity and diminishing their systemic toxicity. These vehicles present advantages not just for peptides but also for small chemical compounds, allowing the control of their release at the desired site. Moreover, the property of polymer multivalency allows the conjugation of several active compounds within the same polymeric carrier, the combination of doxy and other drug in the same polymer carrier would synergistically enhance the therapeutic value of these prodrugs. Synergy could also be effective when conjugates of each drug are administered per separate [18]. Due to the molecular complexity of FAP and AD it is expected to achieve better therapeutic output if more than one molecular pathway of disease is targeted.

### SUMMARY OF THE INVENTION

The present invention, in some embodiments thereof, relates to novel chemical conjugates and to uses thereof in therapy and diagnosis and, more particularly, but not exclusively, to novel conjugates of polymers having attached thereto one or more therapeutic agents and optionally a targeting moiety and/or a labelling probe, for treatment and diagnosis of amyloidosis related diseases.

In particular, the present invention relates to a polymer-drug conjugate where the polymeric backbone bears, at least, a fibrillar disrupting agent selected from the group including: anthracyclines antibiotics like the tetracycline, rolitetracycline, minocycline and /or doxycycline or their derivates, etc,...able to disaggregate or break the amyloid fibrils and/or block the aggregates. This conjugate may contain also in its structure targeting moieties which allow an effective targeting of the polymer-drug conjugate to the diseased area, providing higher efficacy in the treatment and/or diagnosis of amyloidosis related diseases, including polyneuropathic disorders and neurodegenerative diseases like FAP and AD.

Polymer-drug conjugates of the present invention have shown a marked specificity retaining or enhancing the inherent activity of the selected bioactive molecules, allowing, thanks to the multivalency of the polymers, the obtaining of synergistic effects through the combination therapy, higher stability and lower side toxicity of the parent drug achieving a better therapeutic index.

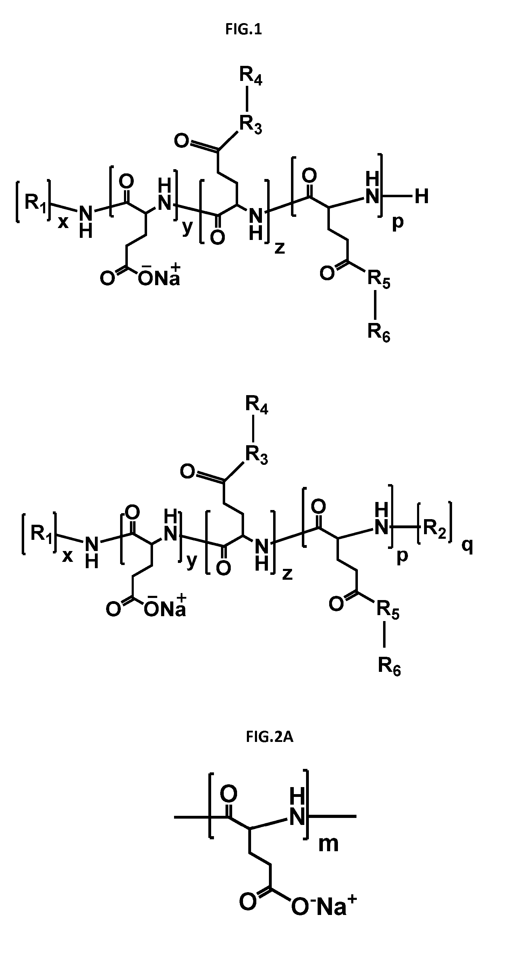

Therefore, the present invention provides a polymer-drug conjugate represented in the general formula I, also represented in Figure 1:

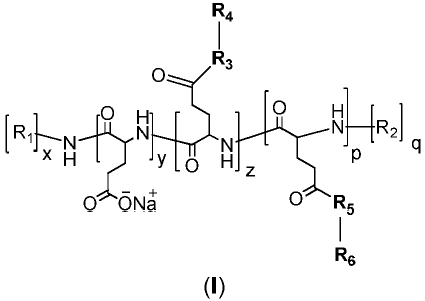

Wherein:
R₁ represent an alkyl group, defined C-terminal linking point (alkyne, azide, thiol, activated thiols, halides, alkenes, activated esters, activated alcohols, protected amines, maleimide group, acetals, activated carboxylic groups), ethylenglycol (EG) of different molecular weights including poly(ethylene glycol) (PEG from 100 to 20000g/mol).
R₂ represents a hydrogen atom, an alkyl group, defined C-terminal linking point (alkyne, azide, thiol, activated thiols, halides, alkenes, activated esters, activated alcohols, protected amines, maleimide group, acetals, activated carboxylic groups), ethylenglycol (EG) of different molecular weights including poly(ethylene glycol) (PEG from 100 to 20000g/mol), PEG-thiol, PEG-4TP.
R₃ represents the linking spacer or bond between the polymer main chain and the bioactive agent (R₄) as itself or derivated and is an alkyl group, defined C-terminal linking point (alkyne, azide, thiol, activated thiols, halides, alkenes, activated esters, activated alcohols, protected amines, maleimide group, acetals, activated carboxylic groups), ethylenglycol (EG) of different molecular weights including poly(ethylene glycol) (PEG from n=2-16), aminoacids such as lysine, arginine, imidazol, histidine, cysteine and secondary and tertiary amino groups and aminoacid sequences.
R₄ is the selected drug for amyloidosis treatment, selected from the group which comprises the anthracycline antibiotics such as tetracycline, rolitetracycline, minocycline, doxycycline and their derivatives, and the drug can be covalent linked to the polymer chain as itself or previously derivatised (including the introduction of amine, thiol, carbonyl, vinyl, alcohol or carboxyl groups).
R₅ represents the linking spacer or bond between the polymer main chain and the bioactive agent R₆ as itself or derivatised and is an alkyl group, defined C-terminal linking point (alkyne, azide, thiol, activated thiols, halides, alkenes, activated esters, activated alcohols, protected amines, maleimide group, acetals, activated carboxylic groups), ethylenglycol (EG) of different molecular weights including poly(ethylene glycol) (PEG from n=2 to n=16), aminoacids such as lysine, arginine, imidazol, histidine, cysteine and secondary and tertiary amino groups and aminoacid sequences.
R₆ is the second selected drug for amyloidosis treatment, selected from the group which comprises the anthracycline antibiotics such as tetracycline, rolitetracycline, minocycline, doxycycline and their derivatives (the drug can be covalent linked to the polymer chain as itself or previously derivated); or a labelling moiety exploited for conjugate monitoring, for biodistribution experiments or as a diagnostic probe where the labelling agent comprises fluorescent probes for optical imaging such as Cy5.5, coordination complexes for MRI, or tracers for PET and SPECT, including the chelating agents DTPA, DOTA, NOTA, NODA and metallic ligands such as gallium, technetium, gadolinium, indium.
x is the monomer units included in R₁, from 1 to 1000.
y is an integer having a value such that y/(x+y+z+p+q) multiplied by 100 is in the range of from 0.01 to 99.9
z is an integer having a value such that z/(x+y+z+p+q) multiplied by 100 is in the range of from 0.01 to 99.9
p is an integer having a value such that z/(x+y+z+p+q) multiplied by 100 is in the range of from 0.01 to 99.9
q is the monomer units included in R₂, from 1 to 1000.
R2, R3 and R5 can be used for conjugation of bioactive agents (including low molecular weight drugs, peptides, proteins, antibodies), near infrared dyes, coordination complexes for MRI, PET and SPECT tracers.

In addition, the present invention provides pharmaceutical compositions which comprise the conjugates herein described with a pharmaceutically acceptable carrier.

The polymer-drug conjugate is for use in a method of treating or diagnosing amyloidosis in a subject in need of thereof, the method comprising administering to the subject a therapeutically effective amount of the polymer-drug conjugate as described herein.

### FIGURES

Figure 1: General formula I.
Figure 2A: PGA chemical structure.
Figure 2B: Doxy chemical structure.
Figure 2C: Doxy-NH₂ chemical structure.
Figure 2D: Bz-Gly-Gly-CONH-Doxy chemical structure.
Figure 2E: Bz-Leu-Gly-CONH-Doxy chemical structure.
Figure 2F: Gly-Gly-CONH-Doxy chemical structure.
Figure 2G: Leu-Gly-CONH-Doxy chemical structure.
Figure 2H: PGA-CONH-Doxy chemical structure.
Figure 2I: PGA-COO-Doxy chemical structure.
Figure 2J: PGA-CONH-AA-Doxy chemical structure.
Figure 2K: Doxy-NH-Gly-Leu chemical structure.
Figure 2L: Doxy-NH-Gly-Gly chemical structure.
Figure 2M: PGA-DOXYchemical structure.
Figure 3: Doxycycline chemical derivatisation reaction.
Figure 4: Synthesis of the conjugate PGA-CONH-Doxy. Method A (NHS/DMAP).
Figure 5: Synthesis of the conjugate PGA-CONH-Doxy. Method B (DIC/HOBt).
Figure 6: Synthesis of the conjugate PGA-CONH-Doxy. Method C (DMTMM coupling).

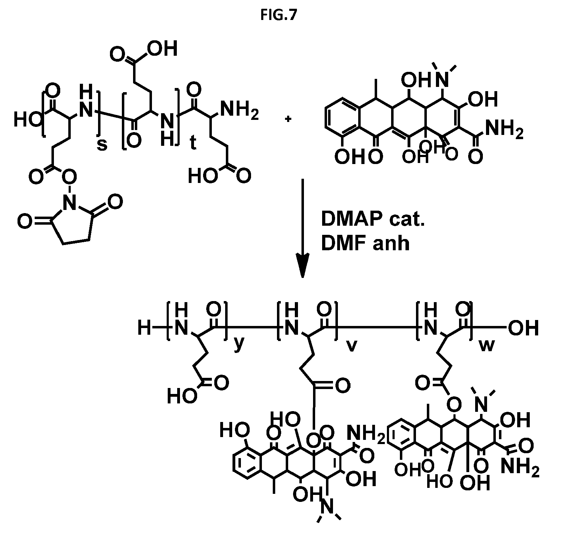

Figure 7: Synthesis of PGA-COO-Doxy.
Figure 8: Synthesis of PGA-CONH-AA-Doxy.
Figure 9: NMR-¹H analysis (300Hz, MeOD) of the amino-derivatised doxycycline (doxy-NH₂).
Figure 10: MS-MALDI TOF analysis of the amino-derivatised doxycycline (doxy-NH₂).
Figure 11: Example of a PGA-Doxy GPC trace (absorbance at 273nm, mobile phase: PBS pH7.4, tR=12min)
Figure 12: Doxycycline analysis by LCMS. (A) UPLC spectra (B) Mass Spectroscopy spectra (scan mode).
Figure 13: Doxycycline release from the conjugates under hydrolytical conditions in PBS at different pHs at 37°C. The area under the curve (AUC) obtained by HPLC (abs.273nm) is represented in Y axis like percentage of the initial area of the conjugate.
Figure 14: Stability of PGA-X-Doxy conjugates in *in vitro* conditions. (A) HPLC representation of the area under the curve (AUC) vs. the incubation time (days) in PBS at 37°C. (B) Example of the HPLC chromatograms obtained analysing the aliquots of one conjugate.
Figure 15: SANS diagram of the PGA-CONH-Doxy conjugates solutions in deuterated PBS (A) graphic, (B) numerical data (r=radius, L=length).
Figure 16: Effect of Doxycycline conjugates was tested on the *in vitro* FAP model. In the control situation, after 13 days incubation, TTR initial aggregates are organised into mature fibrils.(A) When doxy conjugates were active (i.e. PGA-CONH-Doxy conjugates), fibrils were disrupted producing small round particles and shorter fibrils. (B) When doxy concentration was increased, activity was observed. (C) Doxy-NH2 showed comparable activity to the original parent drug.
Figure 17: Particle size (radious (nm)) measured by Dynamic Light Scattering. (* significant data respect control (t=3days), # significant data respect control (t=6days), p<0.05).
Figure 18: Haemolytic activity of PGA-CONH-Doxy. Data expressed as mean (n=3).
Figure 19: PGA-CONH-Doxy labelled with the fluorescence probe Cy5.5.
Figure 20: Biodistribution of the conjugate PGA-CONH-Doxy-Cy5.5. *Ex vivo* analysis of the organs after 4/24h post-administration by optical imaging with IVIS®.
Figure 21: Biodistribution of the conjugate PGA-CONH-Doxy-Cy5.5. Fluorescent signal quantification after homogenisation of the excised organs.
Figure 22: Immunohistochemistry analysis to evaluate the non-fibrilar deposition of the TTR in determined organs (liver, intestine, oesophagus and stomach) in the treated animals (group B: control animals with disease, group A: treated animals with PGA-CONH-Doxy conjugate, group D (combination group): treated animals with a PGA-CONH-Doxy conjugate and a TTR aggregates-blocking agent. Semi-quantitative representation of the results.

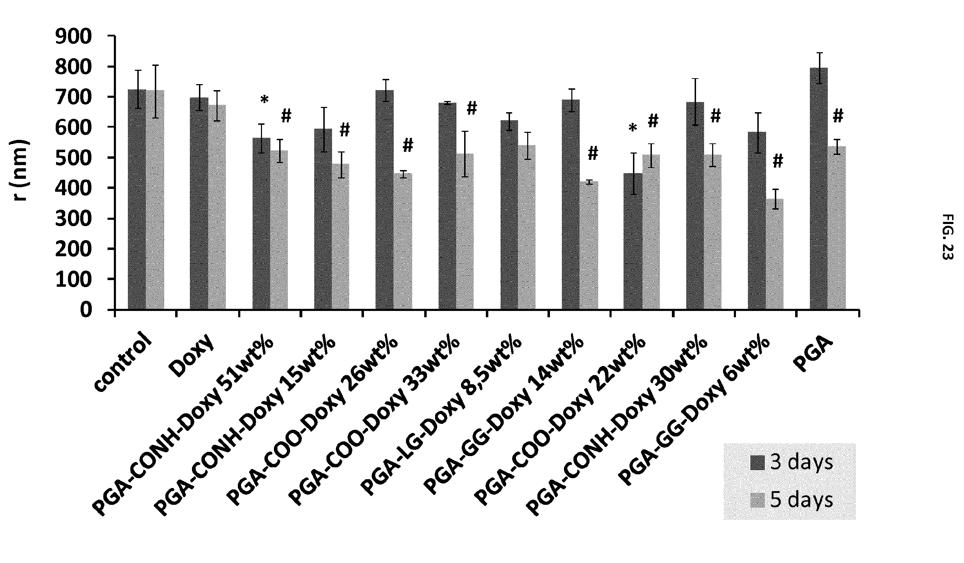

Figure 23: Examples of histology images (haematoxylin/eosin staining) from sliced and fixed organs (liver and intestine (I1/I4).

### DESCRIPTION OF THE INVENTION

The present invention, in some embodiments thereof, relates to novel chemical polymer-drug conjugates for use in therapy and, more particularly, but not exclusively, to novel conjugates of polymers having attached thereto a fibril disrupter agent and/or a aggregate-blocking agent and/or a targeting moiety and/or labelling moiety and to uses thereof in treating amyloidosis. The present invention relates to a polymer-drug conjugate where the polymeric backbone bears, at least, a fibrillar disrupting agent able to disaggregate or break the amyloid fibrils and/or block the aggregates. This conjugate may contain also in its structure targeting moieties which allow an effective targeting of the polymer-drug conjugate to the diseased area, providing higher efficacy in the treatment and/or diagnosis of amyloidosis.

This invention provides novel polymer-drug conjugates and pharmaceutical compositions which contain them for use as pharmaceutical agents against diseases involving amyloidosis, including amyloidosis related to polyneuropathic disorders and neurodegenerative disorders like FAP and AD.

In one embodiment of this invention the polymer-drug conjugates where the polymeric backbone bears, at least, a bioactive agent for amyloidosis treatment and at least a targeting moiety and a labelling probe, are for use in diagnosis and to perform biodistribution studies of the bioactive agent in animal models with amyloidosis related diseases.

The present invention relates to polymer-drug conjugates which comprise a polymeric backbone to which is covalent linked at least a bioactive agent, with the capability of disrupting amyloid fibrils and /or blocking aggregates activity.

The phrase "amyloid disrupter/disrupting agent", as used herein, describes any therapeutic agent that directly disaggregates or breaks the amyloid fibrils and/or their deposits and promotes a concatenate effect improving disease conditions as observed by amyloid markers studies.

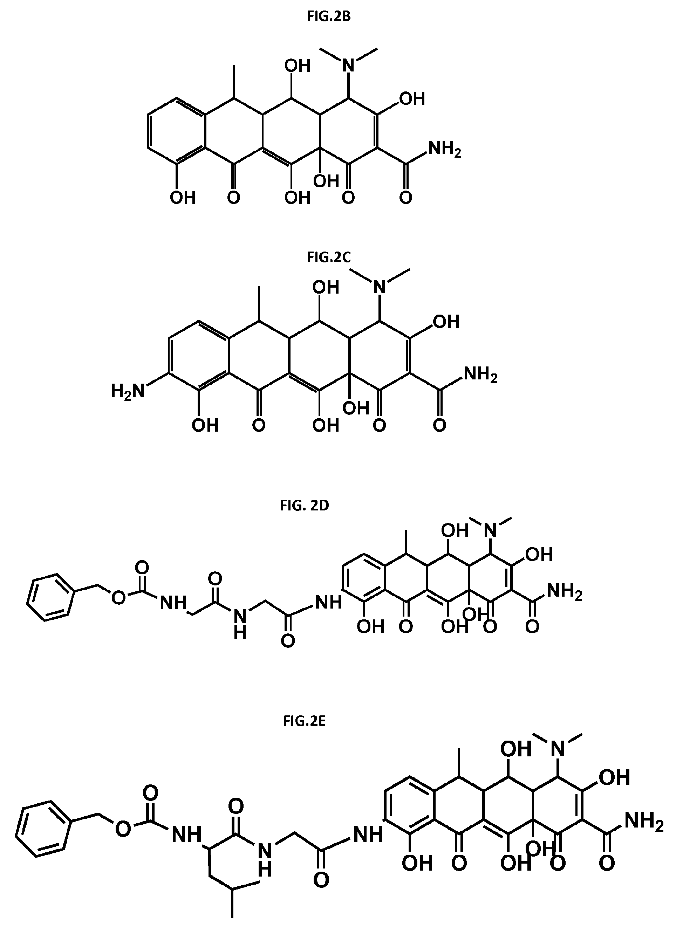

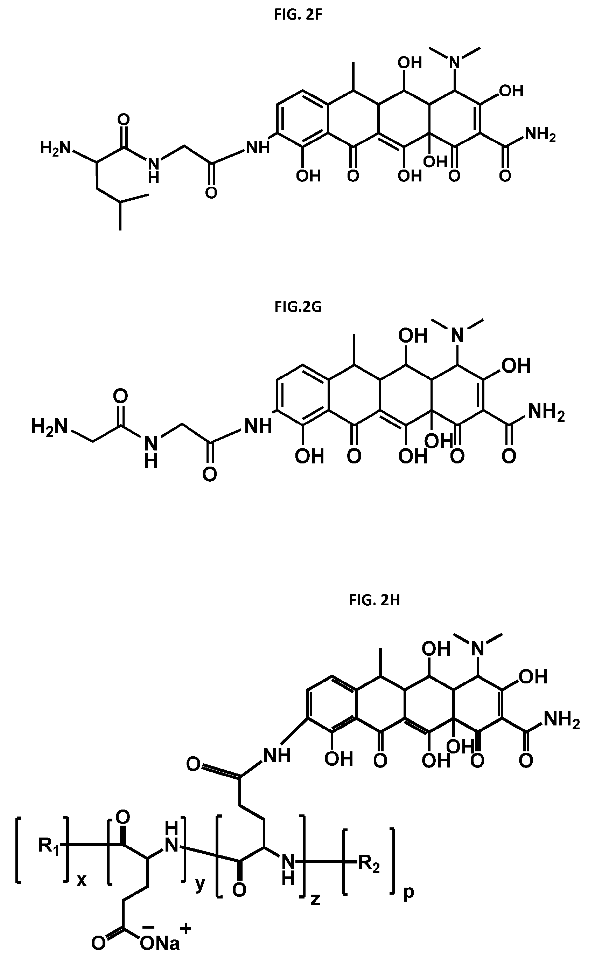

Exemplary amyloid disrupting agents include, anthracyclines antibiotics such as tetracycline, rolitetracycline, minocycline, and/or doxycycline and their derivates, being preferable the use of doxycycline, as it is represented in the Fig.2B, and its derivate Doxy.-NH₂.
The phrase "aggregates-blocking agent", as used herein, describes any therapeutic agent that directly interacts with the misfolded protein in its aggregate stage being able to avoid interaction with the receptor, thus avoiding cell death cascades.

In some embodiments of the present invention, the described conjugates comprise a polymeric backbone that represents the total plurality of units of the main chain, from which, some of these units are linked to the first therapeutic agent, optionally other ratio of these units is linked to the second bioactive agent, and optionally other ratio has been labelled with a probe for the monitoring of the conjugate; and optionally some of the units of the polymeric backbone remain free and other may be linked to a targeting moiety to allow the whole system to cross the blood-brain barrier.

The bioactive agent or the bioactive agents are linked to the polymeric matrix directly or through a spacer or linker, being this linker biodegradable and selected from the group consisting of a pH-sensitive and an enzymatically-cleavable linker. In the case of enzymatic linkages, these are cleaved as a consequence of the presence of enzymes over-expressed in inflamed areas.

The union comprises a direct covalent bonding of the active agent, via amide, ester, acetal, hydrazone or disulphide bond; and it may include any amino acid sequence including those substrates of metalloprotases (i.e. MMP-9 (PVGLIG)), cathepsin B (GPLG; R; PL) and other peptide sequences able to be recognised and cleaved in presence of enzymes located in drug release area. Preferably, the secuences Gly-Gly and Leu-Gly are used in the present invention.

According to some embodiments of the invention, the polymeric backbone is derived from a polyglutamic acid (PGA). According to some embodiments of the invention, the polymeric backbone is derived from polyglutames such as block-co-polymers, triblocks, where the other blocks include without limitation polyethylenglycol (PEG), water soluble polyamino acid, polylactic acid (PLA), polylactic-co-glycolic acid (PLGA), poly(D,L-lactide-co-glycolide) (PLA/PLGA), poly(hydroxyalkylmethacrylamide), polyglyceron, polyamidoamine (PAMAM) and polyethyleneimine (PEI), and diferent structures of the polyglutamic acid (PGA) represented in the Fig. 2A, e.g. star PGA, brush PGA,etc.) being preferable the use of poly-L-glutamic acid, poly-D-glutamic acid or poly-D,L-glutamic acid.

The present patent includes all the possible polymer-drug linkages derived from PGA modification of its pendant chains (e.g. alkyne, azide, tiol, halides, activated esters, activated alcohols, protected amines, maleimide groups, acetals, ethylene glycol (EG) of several molecular weights including polyethyleneglycol (PEG from 100 to 20000g/mol) as well as drug modification for achieving all the correspondent type of bond with the polymer chain, directly or through spacers. All the possible combinations are included in this invention.

Optionally, the polymer-drug conjugate of the present invention could contain a targeting moiety able to cross specific biological barriers, for example the blood-brain barrier (BBB). This targeting residue can be selected from the group which comprises peptide, proteins o a monoclonal antibody. Examples of targeting residues are any ligand capable to target the transferrin receptor (e.g. the transferrin protein (holo- and apo-transferrin), monoclonal antibodies (e.g. the monoclonal (mAb) OX26), the cyclic peptide CRTIGPSVC, etc.) or to target other receptor present in the BBB such as de low density lipoprotein receptor-related protein 1 (LRP-1) like the peptide Angiopep2.

According to some embodiments of the present invention it is considered the incorporation of a labelling probe such as a near infrared dye for optical imaging, coordination complexes for MRI, and tracers for PET and SPECT studies. The use of Cy5.5 as probe is preferred in the present invention.

As used herein, the term "mol%" describes the number of moles of an attached moiety per 1mol of the polymeric conjugate, multiplied by 100. Thus, i.e. a 1mol% load of a fibril disrupting agent describes a polymeric conjugate composed of 100 backbone units, whereby 1 backbone unit has the bioactive agent attached thereto and the other 99 units are either free or have other agents attached thereto.

The optimal degree of loading of the therapeutically active agent (or agents) and the targeting moiety for a given conjugate and a given use is determined empirically based on the desired properties of the conjugate (e.g. water solubility, therapeutic efficacy, pharmacokinetic profile, toxicity and dosage requirements), and optionally on the amount of the conjugated moiety that can be attached to a polymeric backbone in a synthetic pathway of choice.

According to some embodiments of the invention, the drug loading attached to the water soluble polymer can vary. In general, the polymer-drug conjugates are characterized by a load of the therapeutically active agent or the targeting moiety greater than 0.5 mol%.

In certain aspects of the invention, the amount of fibril disrupter drug conjugated per water-soluble polymer can vary. At the lower end, such a composition may comprise from about 1%, about 2%, about 3%, about 4%, about 5%, about 6%, about 7%, about 8.degree./a, about 9%, or about 10%, about 11%, about 12%, about 13%, about 14%, about 15%, about 16%, about 17%, about 18%, about 19%, about 20%, about 21% about 22%, about 23%, about 24%, to about 25% (w/w) fibril disrupter drug relative to the mass of the conjugate. At the high end, such a composition may comprise from about 26%, about 27%, about 28%, about 29%, about 30%, about 31% about 32%, about 33%, about 34%, about 35%, about 36%, about 37%, about 38%, about 39%, to about 40% or more (w/w) fibril disrupter drug relative to the mass of the conjugate.

Therefore, the present invention provides a polymer-drug conjugate represented in the general formula I, also represented in Figure 1:

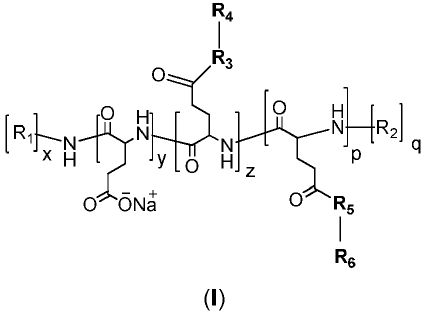

Wherein:
R₁ represent an alkyl group, defined C-terminal linking point (alkyne, azide, thiol, activated thiols, halides, alkenes, activated esters, activated alcohols, protected amines, maleimide group, acetals, activated carboxylic groups), ethylenglycol (EG) of different molecular weights including poly(ethylene glycol) (PEG from 100 to 20000g/mol).
R₂ represents a hydrogen atom, an alkyl group, defined C-terminal linking point (alkyne, azide, thiol, activated thiols, halides, alkenes, activated esters, activated alcohols, protected amines, maleimide group, acetals, activated carboxylic groups), ethylenglycol (EG) of different molecular weights including poly(ethylene glycol) (PEG from 100 to 20000g/mol), PEG-thiol, PEG-4TP.
R₃ represents the linking spacer or bond between the polymer main chain and the bioactive agent (R₄) as itself or derivated and is an alkyl group, defined C-terminal linking point (alkyne, azide, thiol, activated thiols, halides, alkenes, activated esters, activated alcohols, protected amines, maleimide group, acetals, activated carboxylic groups), ethylenglycol (EG) of different molecular weights including poly(ethylene glycol) (PEG from n=2-16), aminoacids such as lysine, arginine, imidazol, histidine, cysteine and secondary and tertiary amino groups and aminoacid sequences.
R₄ is the selected drug for amyloidosis treatment, selected from the group which comprises the anthracycline antibiotics such as tetracycline, rolitetracycline, minocycline, doxycycline and their derivatives, and the drug can be covalent linked to the polymer chain as itself or previously derivatised (including the introduction of amine, thiol, carbonyl, vinyl, alcohol or carboxyl groups).
R₅ represents the linking spacer or bond between the polymer main chain and the bioactive agent R₆ as itself or derivatised and is an alkyl group, defined C-terminal linking point (alkyne, azide, thiol, activated thiols, halides, alkenes, activated esters, activated alcohols, protected amines, maleimide group, acetals, activated carboxylic groups), ethylenglycol (EG) of different molecular weights including poly(ethylene glycol) (PEG from n=2 to n=16), amino acids such as lysine, arginine, imidazol, histidine, cysteine and secondary and tertiary amino groups and amino acid sequences.
R₆ is the second selected drug for amyloidosis treatment, selected from the group which comprises the anthracycline antibiotics such as tetracycline, rolitetracycline, minocycline, doxycycline and their derivatives (the drug can be covalent linked to the polymer chain as itself or previously derivated); or a labelling moiety exploited for conjugate monitoring, for biodistribution experiments or as a diagnostic probe where the labelling agent comprises fluorescent probes for optical imaging such as Cy5.5, coordination complexes for MRI, or tracers for PET and SPECT, including the chelating agents DTPA, DOTA, NOTA, NODA and metallic ligands such as gallium, technetium, gadolinium, indium.
x is the monomer units included in R₁, from 1 to 1000.
y is an integer having a value such that y/(x+y+z+p+q) multiplied by 100 is in the range of from 0.01 to 99.9
z is an integer having a value such that z/(x+y+z+p+q) multiplied by 100 is in the range of from 0.01 to 99.9
p is an integer having a value such that z/(x+y+z+p+q) multiplied by 100 is in the range of from 0.01 to 99.9
q is the monomer units included in R₂, from 1 to 1000.

R2, R3 and R5 can be used for conjugation of bioactive agents (including low molecular weight drugs, peptides, proteins, antibodies), near infrared dyes, coordination complexes for MRI, PET and SPECT tracers.

The compounds of the present invention may contain one or more basic nitrogen atoms and, therefore they may form salts with acids that also form part of this invention. Examples of pharmaceutically acceptable salts include, among others, addition salts with inorganic acids such as hydrochloric, hydrobromic, hydroiodic, nitric, perchloric, sulphuric and phosphoric acid, as well as addition of organic acids as acetic, methanesulfonic, trifluoromethanesulfonic, ethanesulfonic, benzenesulfonic, p-toluenesulfonic, benzoic, camphorsulfonic, mandelic, oxalic, succinic, fumaric, tartaric, and maleic acid. Likewise, compounds of the present invention may contain one or more acid protons and, therefore, they may form salts with bases, which also form part of this invention. Examples of these salts include salts with metal cations, such as for example an alkaline metal ion, an alkaline-earth metal ion or an aluminium ion; or it may be coordinated with an organic with an organic or inorganic base. An acceptable organic base includes among others diethylamine and triethylamine. An acceptable inorganic base includes aluminium hydroxide, calcium hydroxide, potassium hydroxide, sodium carbonate, and sodium hydroxide.

Salts derived from pharmaceutically acceptable organic nontoxic bases include salts of primary, secondary, and tertiary amines, substituted amines including naturally occurring substituted amines, cyclic amines, and basic ion exchange resins, such as arginine, betaine, caffeine, choline, N,N-dibenzylethylenediamine, diethylamine, 2-diethylaminoethanol, 2-dimethylaminoethanol, ethanolamine, ethylenediamine, N-ethylmorpholine, ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and similar ones.

There is no limitation on the type of salt that can be used, provided that these are pharmaceutically acceptable when they are used for therapeutic purposes. Salts can be synthesized from the parent compound which contains a basic or acidic moiety by conventional chemical methods. In general, such salts can be prepared by reaction of the free acid or base forms of these compounds with a stochiometric amount of the appropriate base or acid in water or in an organic solvent, such as ether, ethyl acetate, ethanol, isopropanol, or acetonitrile or in a mixture of them.

Some of the compounds of **formula I** of the present invention may exist in unsolvated as well as solvated forms such as, for example, hydrates. The present invention encompasses all such above-mentioned forms which are pharmaceutically active.

Some of the compounds of general **formula I** may exhibit polymorphism, encompassing the present invention all the possible polymorphic forms, and mixtures thereof.
Various polymorphs may be prepared by crystallization under different conditions or by heating or melting the compound followed by gradual or fast cooling. The presence of polymorphs may be determined by solid NMR spectroscopy, IR spectroscopy, differential scanning calorimetry, powder X-ray diffraction or such other techniques. Another aspect of the present invention relates to a process for the preparation of polymer conjugate compounds of **formula I,** their derivatives, their analogues, their tautomeric forms, their stereoisomers, their polymorphs or their pharmaceutical acceptable salts and solvates.

According to some embodiments of the invention there is provided a pharmaceutical composition, comprising, as an active ingredient, the conjugate as described herein and a pharmaceutically acceptable carrier.
According to some embodiments of the invention, the composition is being packaged in a packaging material and identified in print, in or on the packaging material for use in the treatment of a medical condition associated with amyloidosis.

The polymer-drug conjugate is for use in a method of treating amyloidosis in a subject in need of thereof, the method comprising administering to the subject a therapeutically effective amount of the conjugate as described herein.

Alternatively, the polymer-drug conjugate is for use in a method of monitoring the conjugate within a body of a patient, the method comprising: administering to the patient the conjugate having a labelling agent as described herein attached thereto; and employing an imaging technique for monitoring a distribution of the conjugate within the body.

The compounds of the present invention can be administered in the form of any pharmaceutical formulation. The pharmaceutical formulation will depend upon the nature of the active compound and its route of administration. Any route of administration may be used, for example such as oral, buccal, pulmonary, topical, parenteral (including subcutaneous, intramuscular, and intravenous), transdermal, ocular (ophthalmic), inhalation, intranasal, otic, transmucosal, implant or rectal administration. However oral, intranasal or parenteral administration are preferred. For the preparation of any of the different formulations included in the present description, it would be used the techniques and methods known in the state-of-the-art, as well as it would be selected the most common excipients utilized in pharmacy for the preparation of the different formulations.

Solid compositions for oral administration include among others tablets, granulates and hard gelatin capsules, formulated both as immediate release or modified release formulations.

Alternatively, the compounds of the present invention may be incorporated into oral liquid preparations such as emulsions, solutions, dispersions, suspensions, syrups, elixirs or in the form of soft gelatin capsules. They may contain commonly-used inert diluents, such as purified water, ethanol, sorbitol, glycerol, polyethylene glycols (macrogols) and propylene glycol. Aid compositions can also contain coadjuvants such as wetting, suspending, sweetening, flavouring agents, preservatives, buffers, chelating agents and antioxidants.
Injectable preparations for parenteral administration comprise sterile solutions, suspensions or emulsions in oily or aqueous vehicles, and may contain coadjuvants, such as suspending, stabilizing, tonicity agents or dispersing agents.
The compound can also be formulated for its intranasal application. Formulations include particles, powders, solutions wherein the compound is dispersed or dissolved in suitable excipients. In one embodiment of the invention the pharmaceutical composition is in the form of nanospheres, microparticles and nanoparticles.
The effective dosage of active ingredient may vary depending on the particular compound administered, the route of administration, the nature and severity of the disease to be treated, as well as the age, the general condition and body weight of the patient, among other factors. A representative example of a suitable dosage range is from about 0.001 to about 100 mg/Kg body weight per day, which can be administered as single or divided doses. However, the dosage administered will be generally left to the discretion of the physician.
As used therein the term "treatment" includes treatment, prevention and management of such condition. The term "pharmaceutically acceptable" as used herein refers to those compounds, compositions, and/or dosage forms which are, within the scope of medical judgement, suitable for use in contact with the tissues of humans and animals without excessive toxicity, irritation, allergic response, or other problem or complication, commensurate with a reasonable benefit/risk ratio.

According to an aspect of embodiments of the invention there is provided a process of synthesizing the conjugate described herein, the process comprising:
(a) Co-polymerising a plurality of monomeric units of the polymer, at least one of the monomeric units termination by a first reactive group, and at least one of the monomeric units terminating by a second reactive group, to thereby obtain a copolymer that comprises a plurality of backbone units, at least one backbone unit having the first reactive group and at least one backbone unit having the second reactive group, the first reactive group being capable of reacting with the targeting moiety and the second reactive being capable of reacting with the therapeutically active agent, or:
(b) Polymerisation of a single monomeric unit by means of a polymer block as initiator, and/ or
   Post-modification after polymerisation of the first polymeric synthesised block achieving two or more different reactive ending groups in the starting polymer chain with modifiable polymer lengths to the original backbone, giving different final polymeric structures and /or conformations in solution, and/or
   Construction of a triblock polymer based structure where the block in between posses the first reactive group being capable of reacting with the therapeutical agent(s) and/or the labelling agent and one of the ending polymeric blocks terminates by a second reactive group being capable of reacting with the labelling agent, the targeting moiety or a second therapeutic agent.
(c) Reacting the polymer carrier with the targeting moiety or a derivative thereof, via the first reactive group, to thereby obtain a polymeric vehicle having the targeting moiety attached to a polymeric backbone thereof; and
(d) Reacting the polymer carrier with the therapeutically active agent or a derivative thereof, via the second reactive group, to thereby obtain the polymer vehicle having the therapeutically active agent attached to a polymeric backbone thereof, thereby obtaining the conjugate of formula I or reacting the polymer carrier with the first therapeutically active agent or a derivative thereof, and secondly the next therapeutically agent (already linked to another polymer or to be linked to the post-modified initial polymer) to the main polymer chain.

According to some embodiments of the invention, step (b) is performed subsequent to, concomitant with or prior to (c).

According to some embodiments of the invention, at least one of the monomer units terminating by the first or the second reactive group further comprises a linker linking the reactive group to the monomeric unit.

Unless otherwise defined, all technical and/or scientific terms used herein have the same meaning as commonly understood by one of ordinary skill in the art to which the invention pertains. Although methods and materials similar or equivalent to those described herein can be used in the practices or testing of embodiments of the invention, exemplary methods and/or materials are described below. In case of conflict, the patent specification, including definitions, will control.

### EXAMPLES

### MATERIALS AND METHODS

All reactions requiring anhydrous conditions were performed under argon or nitrogen atmosphere.

Chemicals and solvents are either A.R. grade or purified by standard techniques.

Size Exclusion chromatography (SEC). Sephadex G25 resin / G10 resin columns, eluent H₂O. SEC analysis was performed using a HPLC system from Waters (Milford, USA), composed by an Autosampler 717, a photodiode array detector unit 2996 (model Z996), 3 pumps 515 and a multi lambda fluorescence detector 2475, with two TSK-gel columns in series (G3000 and G25000 PWXL). A flow rate of 1miL/min and a mobile phase 0.1 M PBS buffer was used. Also for SEC analysis of PEGylated conjugates, Zorbax® column GF-250 (4.6x250mm, 4µm) from Agilent technologies (USA). For reverse phase (RP) chromatography a LIChroCART®, Cat.1.50943 LiChrospher® 100, RP-18 (125 x 4 mm, 5 µm) column purchased from Waters Ltd. (Hertfordshire, UK) was used. As mobile phase, different acetonitrile gradients in aqueous 0.1%TFAwere used. The UV spectra were recorded on Jasco V-630 UV/Vis spectrophotometer.

Nuclear Magnetic Resonance (NMR) analysis ¹H-NRM, ¹³C-NMR and two-dimensional diffusion-ordered NMR spectroscopy (DOSY) were performed with Bruker Advance AC-300 (300MHz) or AV500 (500MHz). The chemical shifts are expressed in δ relative to TMS (δ =0 ppm) and the coupling constants in J in Hz. The spectra are recorded in the deuterated solvent indicated in each case, at 300K.

Liquid Chromatography-Mass Spectrometry (LCMS). Equipment use was an Acquity Ultra Performance LC (Waters) with a PDA detector and a Micromass ZQ Waters 400 LC Single quadrupole mass spectrometer. Chromatography column used was a RP-18 Kinetex (2.6µm x100mm).

Transmission electron microscopy (TEM). Samples were adsorbed to glow-discharged carbon-coated collodion film supported on 200-mesh copper grids. For negative staining, the grids were washed with deionized water and stained with 1% uranyl acetate solution. The grids were visualized with a Zeiss microscope operated at 60 kV. Dynamic Light Scattering (DLS). Particle size was measured using a Malvern Zetasizer Nano ZS instrument, equipped with a 532nm laser at a fixed scattering angle of 90°. Particle Radious (nm) in solution was determined at 37°C for Doxycycline conjugates and in PBS 0.020M PBS at 25°C for RAGE peptide conjugates.

Absorbance and fluorescence detection of *in vitro* or *in vivo* processed samples was performed with the equipment Victor² Wallac 1420 Multilabel HTS Counter Perkin Elmer (Northwolk, CT, EEUU) using the corresponding 96-well plates. Excitation wavelength was 595nm and emission 680nm.

Optical *in vivo* imaging. For fluorescence biodistribution studies, Xenogen IVIS®Spectrum platform was exploited with the Living Image®3.2 software (Caliper Life Sicences, Hopkinton, MA).

Small-angle neutron scattering (SANS) experiments were performed on the D11 instrument at the Institute Laue-Langevin in Grenoble (France). Scattering data are expressed in terms of the scattering vector, Q, which is given by Q=4π/λ·sin(θ/2) were λ is wavelength and θ the angle at which the neutrons are scattered. The incident neutron wavelengths were 6±1Å and 12Å, giving accessible Q-ranges of 0.0017 to 0.42Å ⁻¹ using four different sample-detector distances. Sample solutions were prepared at a conjugate concentration of 0.5-2ωt% on a 1g scale in D₂O (pH=5.5, 0.1 M phosphate buffer) and placed in 2mm path length quartz cells, mounted in a sample changer thermostatted at 37°C (± 0.2). These conditions allowed for the study of conjugates at pH, temperature and ionic strengths mimicking those experienced during drug release *in vivo.* Data were corrected for transmission intensity, electronic background and normalised against a flat scatterer according to the standard procedures for the instrument. The obtained scattering profiles I(Q) vs. Q were analysed according to I(Q) ∝ *φ*Vp P(Q) S(Q) + Binc where *φ* is the volume fraction and Vp the particle volume. The FISH modelling suite was used for the analysis [35]. FISH incorporates parameterised form factors, P(Q) and structure factors, S(Q), to describe the dimensions of the scattering particle and inter-particle interactions.

**Ethics Statement:** All animal procedures were performed in compliance with local legislation guidelines and protocols approved by the Institutional Animal care and Use Committee from Centro de Investigación Príncipe Felipe (CIPF, Valencia, Spain) and Instituto de Biologia Molecular e Cellular (IBMC, Porto, Portugal). Body weight was measured once a week.

### ABBREVIATIONS

%ωt = drug loading in weight percent
4TP = 4-thiopyridine
Ab = antibody
ACN = acetonitrile
AD = Alzheimer Disease
ALS = amyotrophic lateral sclerosis
anh. = anhydrous
AUC = area under the curve
BBB = blood brain barrier
Bz = benzyl
Cy = cyane
DCC = N,N'-dicyclohexylcarbodiimide ddH2O = milliQ water
DIC = N,N'Diisopropylcarbodiimide
DIEA = di-isopropyl ethylamine
DLS = dynamic light scattering
DMF = dimethylformamide
DMTMM·Cl = 4-(4,6-dimethoxy[1,3,5]triazin-2-yl)-4-methylmorpholinium chloride
DOTA = 1,4,7-tetrazacyclododecane-1,4,7-10-tetraacetic acid
Doxy = doxycycline
DTPA = N,N-bis(2-(2,6-dioxomorpholino)ethyl)glycine
FAP = familial amyloidotic polyneuropath
FLI = fluorescence imaging
FPLC = fast protein light chromatography
Ga = galium
GA = glutamic acid
Gd = gadolinium
Gl = gastrointestinal tract
Gly = glycine
GPC = gel permeation cromatography
Hb = haemoglobin
HD = Huntington's disease
HOBt = Hydroxybenzotriazole
HPLC = high preasure light cromatography
i.v. = intravenous
IHC = immunohistochemistry
LCMS = liquid chromatography-mass spectrometry
LRP1= low-density lipoprotein receptor-related protein-1
m/z = mass/charge
MALDI TOF = matrix assisted laser desorption/ionization time of flight
MRI = magnetic resonance imaging
MS = mass spectrometry
MS=Multiple Sclerosis
MW = molecular weight
NHS = N-hydroxysuccinimide
NIR = near infrared
NMR = nuclear magnetic resonance
NODA= 1,4,7-triazacyclononane-1,4-diacetate
NOTA= triazacyclononane-1,4,7-triacetic acid
PAMAM = poly(amido amine)
PB = phosphate buffer
PBS = phosphate buffer saline
PD = Parkinson's disease
PDC = polymer drug conjugate
PEG = polyethylene glycol
PEI = poly(ethyleneimine)
PET = positron emission tomography
PGA = poly-L-glutamic acid
PLA= poly(lactic acid)
PLGA= poly(glycolic acid)
PNS = peripheral nervous system
PPC = polymer protein conjugate
PT = polymer therapeutics
RAGE = receptor for advanced glycation end products
RBC = reed blood cells
RP = reverse phase
RP-HPLC = reverse phase high pressure liquid chromatograpy
RT = room temperature
SANS = small angle neutron scattering
SEC = size exclusion chromatography
SPECT = single photon emission computed tomography
TEM = transmision electron microscopy
Tf = transferrin
TFA = trifluoroacetic acid
TfR = transferrin receptor
t_{R} = retention time
TTR = transthyretin
UPCL = ultra performance light chromatography
UV = ultraviolet
v/v= volume/volume

### Example 1: Synthesis of the polymer-drug conjugates

### DERIVATISATION PROCEDURE OF DOXYCYCLINE (Doxy-NH₂)

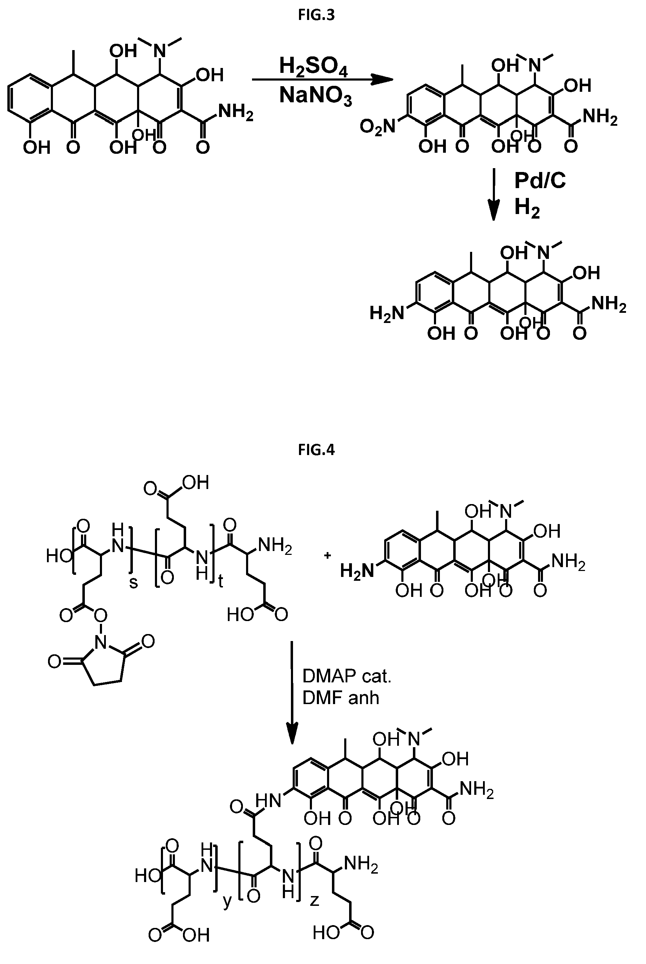

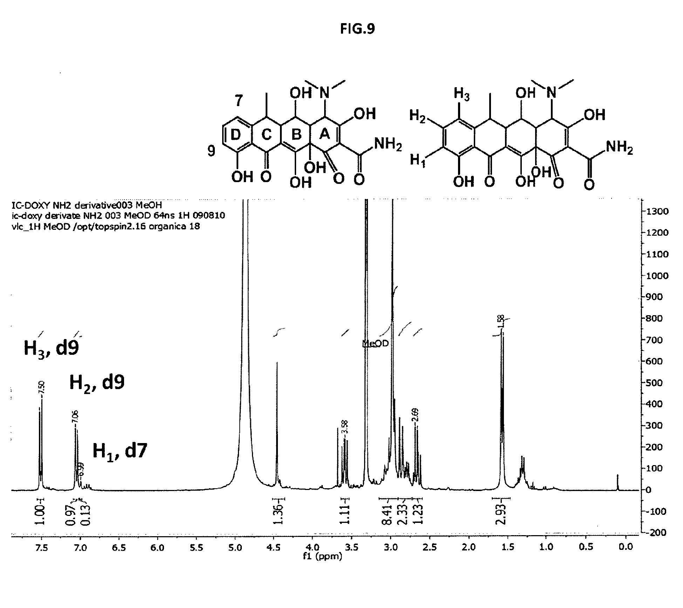

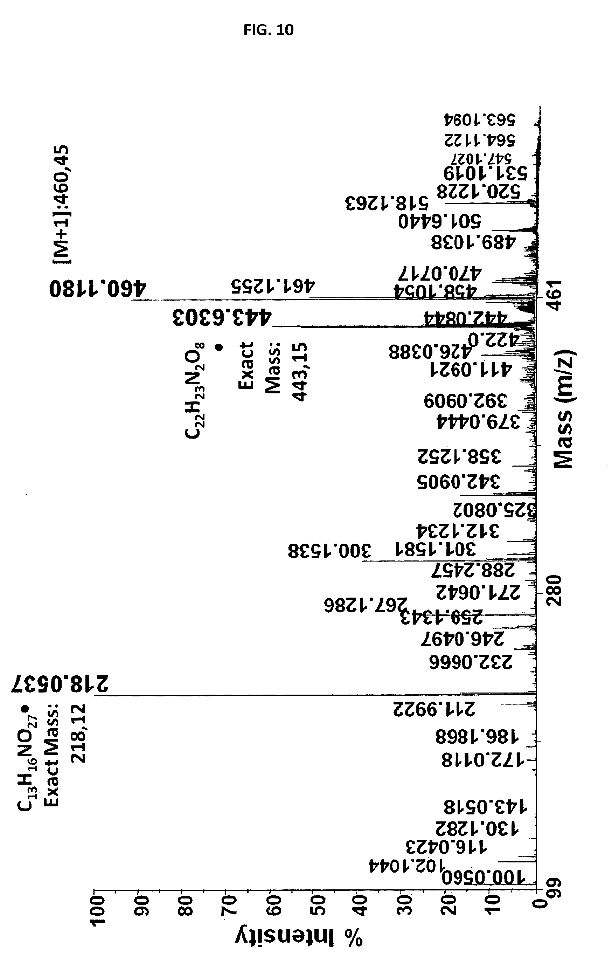

The general derivatisation procedure of doxycycline which results in Doxy-NH₂ obtaining (Compound Figure 2C.) is depicted in Figure 3.
Doxycycline hydrochloride (0.5000g, 512.94g/mol) was slowly added to concentrated H₂SO₄ (1.75mL). After gas evolution had stopped, the orange solution was slowly precipitated into 100mL of cooled diethylether in an ice bath. The hydrosulfate salt was collected by filtration, washed with ether and dried under N₂ flow. The orange powder (542.12g/mol) was redissolved in H₂SO₄ (5mL), cooled to 0°C and NaNO₃ (1.56eq, 101mg, 84.99g/mol) was added over 10min while the reaction was stirring. After 3h at 0°C, reaction was directly precipitated into 200mL of cool diethylether in an ice bath and the mixture was filtered under vacuum. The precipitate was washed with ether and air-dried to give an orange powder which was used without further purification. ¹H-NMR (MeOD) analysis was carried out (Figure 9). Nitration can occur in two different positions (orto-(C9) and para-(C7) positions respect to the hydroxyl group. C9 is occurring preferably. Crude product (0.3634g) was dissolved in 5mL of MeOH and 10%Pd/C (25mg) was added. The system was purged with H₂ and balloon filled with H₂ was used. Reaction was stirred for 2h. After filtration of the catalyst through celite, the solution was diluted with 7.5mL of MeOH and precipitated in 100mL of cold diethylether. Solvent was removed after centrifugation (8000rpm, 4°C, 5min) and pouring off the ether. Orange solid was dried under nitrogen and ¹H-NMR (MeOD) and MS-MALDI TOF analysis were carried out (Figure 10.).
Yield= 70% (derivate in C9 80%). ¹H NMR (300 MHz, MeOD) derivatised in C9 δ 7.57ppm (d, *J* = 8.3 Hz, 1 H), 7.06ppm (d, J = 8.4 Hz, 1 H), derivate in C7 δ 7.15ppm (s, 1H). MS analysis. [M+1]=460.1180

### SYNTHESIS OF PGA-X-Doxy CONJUGATES

Doxycyline conjugation to PGA has been accomplished through different type of linker chemistry, directly to the polymer main chain as well as through cleavable spacers. Synthesis explained below show examples such as ester and amide bonds.

Molar percentage of conjugated drug was varied in all the synthesis described below, in order to obtain a family of conjugates and exploit the different properties (activity, conformation in solution, stability) achieved in each final conjugate. Results vary from 1 to 60w%.
In certain aspects of the invention, the amount of fibril disrupter drug conjugated per water-soluble polymer can vary. At the lower end, such a composition may comprise from about 1%, about 2%, about 3%, about 4%, about 5%, about 6%, about 7%, about 8.degree./a, about 9%, or about 10%, about 11%, about 12%, about 13%, about 14%, about 15%, about 16%, about 17%, about 18%, about 19%, about 20%, about 21% about 22%, about 23%, about 24%, to about 25% (w/w) fibril disrupter drug relative to the mass of the conjugate. At the high end, such a composition may comprise from about 26%, about 27%, about 28%, about 29%, about 30%, about 31% about 32%, about 33%, about 34%, about 35%, about 36%, about 37%, about 38%, about 39%, to about 40% or more (w/w) fibril disrupter drug relative to the mass of the conjugate.

### Synthesis of PGA-COO-Doxy

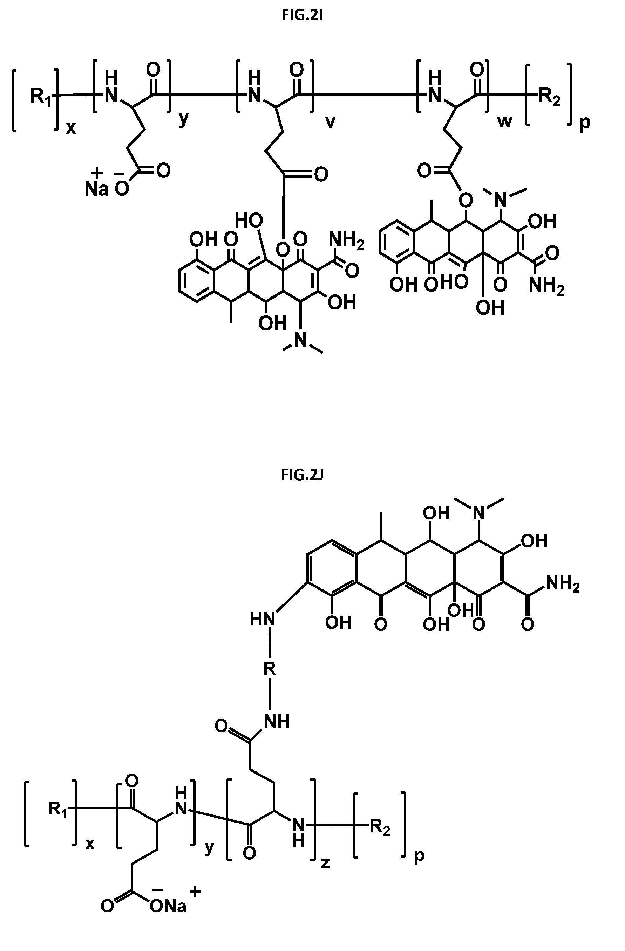

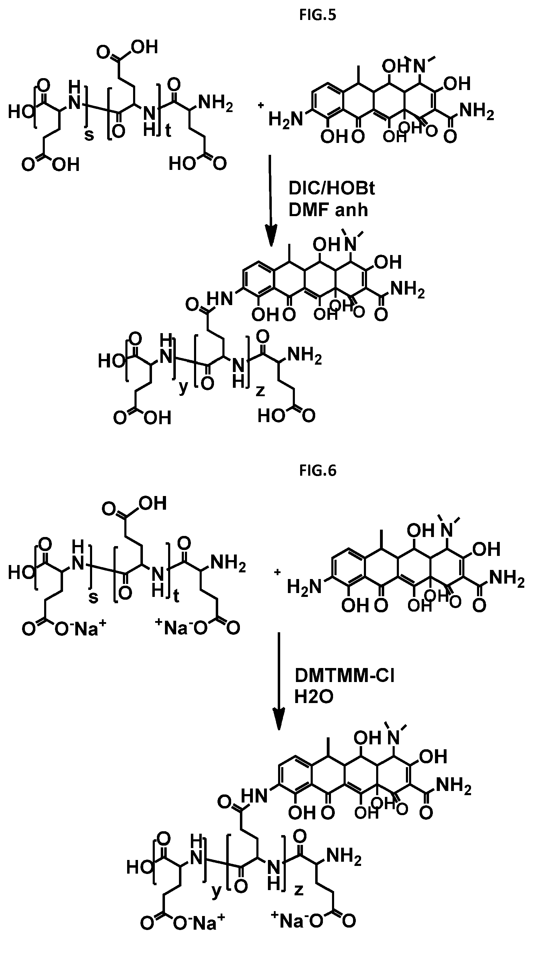

The general synthesis of PGA-COO-Doxy conjugate is depicted in Figure 6. The chemical structures of PGA-COO-Doxy conjugate are presented in Figure 2I. The described methodology explains the linkage of the carboxyl coupling to alcohol groups.

Briefly, carboxyl groups of PGA were previously activated with N-hydroxysuccinimide. PGA-NHS (average MW unit depending on NHS activation, i.e. average MW unit of glutamic acid in case of 52% activation=179.6g/mol) was added to a round bottom flask and dissolved in anhydrous DMF under N₂ atmosphere. Separately, doxy (512.94g/mol) was dissolved in the same conditions. Once dissolved, doxy was dropped in the solution of PGA-NHS. Next, a catalytic amount of DMAP was added and pH was adjusted to 8 with DIEA. After 12-16h under agitation at room temperature, DMF was evaporated under high vacuum until product was dried. Non-reacted drug as well as reaction organic soluble subproducts were removed by washing the crude with a mixture of CHCl₃:acetone (4:1) and placed in ice for 30min. Thereafter, solvent was removed by centrifugation and the solid residue was dried under vacuum.

### Synthesis of PGA-CONH-Doxy

The general synthesis of PGA-CONH-Doxy conjugate is depicted in Figures 4, 5 and 6, depending the method used as explained below. The chemical structure of PGA-CONH-Doxy conjugate is presented in Figure 2H. The described methodology explains the coupling of the carboxyl coupling to an amine group.

### Method A (FIG.4): NHS,DMAP

Previous activated PGA (average MW unit depending on NHS activation, i.e. average MW unit of glutamic acid in case of 52% activation=179.6g/mol) was added to a round bottom flask and dissolved in anhydrous DMF under N₂ atmosphere. Separately, doxy-NH₂ (557.13g/mol) was dissolved in the same conditions. Once dissolved, doxy-NH₂ was dropped in the solution of PGA-NHS. Next, a catalytic amount of DMAP was added and pH was adjusted to 8 with DIEA. After 5h under agitation at room temperature, DMF was evaporated under high vacuum until product was dried. Non-reacted drug as well as reaction organic soluble subproducts were removed by washing the crude with a mixture of CHCl₃:acetone (4:1) and placed in ice for 30min. Thereafter, solvent was removed by centrifugation and the solid residue was dried under vacuum.

### Method B (FIG.5): DIC/HOBt

PGA (average MW unit of glutamic acid=129.1/mol) was added to a round bottom flask and dissolved in anhydrous DMF under N₂ atmosphere. Depending on the molar percentage of drug loading, reagents amount was varied. Next protocol is detailed for 15%mol DIC (0.035mmol, 1.5eq per carboxyl group, 0.836g/cm³, 126.20g/mol) was added to the reaction and after 5min, HOBt (0.035mmol, 1.5eq per carboxyl group, 135.10g/mol) was incorporated as a solid. 10min later, doxy-NH₂ (0.035mmol, 557.13g/mol) dissolved in anhydrous DMF was dropped to the reaction. pH was adjusted to 8 with DIEA. After 12-16h under agitation at room temperature, DMF was evaporated under high vacuum until product was dried. Non-reacted drug as well as reaction organic soluble subproducts were removed by washing the crude with a mixture of CHCl₃:acetone (4:1) and placed in ice for 30min. Thereafter, solvent was removed by centrifugation and the solid residue was dried under vacuum.

### Method C (FIG.6) : DMTMM coupling

Previously, sodium salt form of PGA was placed in a round bottom flask and dissolved in ddH₂O. Separately, and as example for a 30mol% carboxyl group activation doxy-NH₂ (0.6 eq, 557.53g/mol) and DMTMM·Cl (0.3 eq, 276.77g/mol)) were dissolved in also ddH₂O and then added to the flask. pH=8. Reaction was left under agitation 24h at room temperature and protected from light. Then, product was dried by lyophilisation and purified.

### SYNTHESIS OF PGA-CONH-AA-Doxy

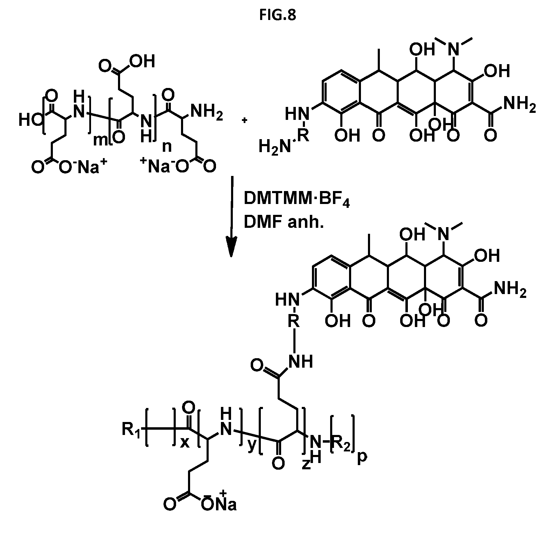

The general synthesis of PGA-CONH-AA-Doxy conjugates (Figure 8.) is depicted in Figure 8. The chemical structures of PGA-COO-Doxy conjugate are presented in Figure 2J. First, the amino-protected peptidil linker (Gly-Gly or Leu-Gly) was first linked to the drug, then deprotected and finally conjugated through an amide bond to the polymeric carrier.

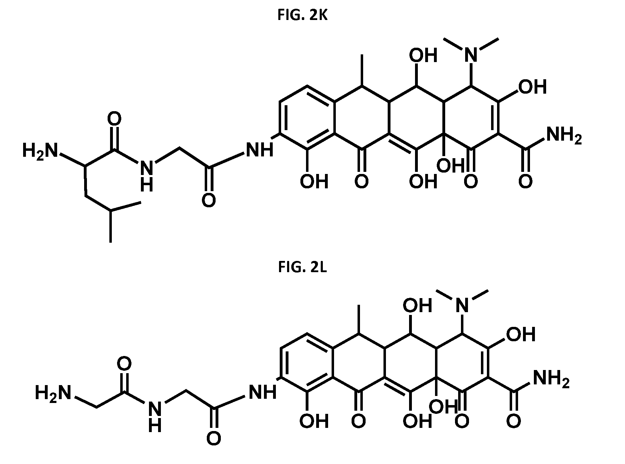

### Synthesis of Leu-Gly-NH-Doxy (Fig. 2K)

Z-Leu-Gly-OH (0.064mmol, 322.36g/mol) was added to a round bottom flask and dissolved in 0.5mL anhydrous DMF under nitrogen atmosphere and continuous agitation. Afterwards, DMTMM·BF₄ (0.07mmol, 1.1 eq, 328.07g/mol) was added dissolved in 0.5mL of anhydrous DMF. After 10minutes, doxy-NH₂ (0.014mmol, 2.2eq, 557.13g/mol) was added dissolved in 2mL of anhydrous DMF. pH was checked to be 8. The reaction was allowed to proceed for 14h stirring at room temperature and protected from light. After this, solvent was removed under vacuum and residue was washed several times with 2mL of ddH₂O through centrifugation (4°C, 4000rpm, 10min). Supernatant was collected and lyophilised. Solid product obtained (28.3mg) was analysed by NMR-¹H and afterwards it was dissolved in 5mL of MeOH for proceeding to remove the protecting group of the amino acid sequence. Solution was placed in a round bottom flask fitted with a septum and a stirring bar. Then a catalytic amount of Pd(OH)₂ Charcoal was added and the flask was purged with N₂ to remove the air and later flask was purged with H₂. Reaction was left stirring for 12h under H₂ pressure. Afterwards, reaction was filtered though a celite column and precipitated over cold diethylether. Ether was removed by centrifugation and the gel obtained was dried over vacuum.

Yield: 70%. ¹H-NMR Doxy-NH-Gly-Leu-Z (300 MHz, MeOD) δ 7.64(m, 1H, C8 Doxy-NH-), 7.40 - 7.08 (m, 5H+1 H C7 Doxy-NH-), 5.19 - 4.93 (m, 2H), 4.14 (dd, J = 9.5, 5.3 Hz, 3H), 4.02 - 3.63 (m, 3H), 1.80 - 1.41 (m, 3H), 0.88 (t, J = 6.5 Hz, 6H),etc.
MS analysis: Doxy-NH-Gly-Leu (deprotected): [M+1]=673.26g/mol.

### Synthesis of Gly-Gly-Doxy (Fig. 2L)

Z-Gly-Gly-OH (0.079mmol, 266.25/mol) was added to a round bottom flask and dissolved in 0.5mL anhydrous DMF under nitrogen atmosphere and continuous agitation. Afterwards, DMTMM·BF₄ (0.087mmol, 1.1eq, 328.07g/mol was added dissolved in 0.5mL of anhydrous DMF. After 10minutes, doxy-NH₂ (0.18mmol, 2.2eq, 557.13g/mol) was added dissolved in 2mL of anhydrous DMF. pH was checked to be 8. The reaction was allowed to proceed for 14h stirring at room temperature and protected from light. After this, solvent was removed under vacuum and residue was washed several times with 2mL of ddH₂O through centrifugation (4°C, 4000rpm, 10min). Supernatant was collected and lyophilised. Solid product obtained (27.1 mg) was analysed by NMR-1 H and afterwards it was dissolved in 5mL of MeOH for proceeding to remove the protecting group of the amino acid sequence. Solution was placed in a round bottom flask fitted with a septum and a stirring bar. Then a catalytic amount of Pd(OH)₂ Charcoal was added and the flask was purged with N₂ to remove the air and later flask was purged with H₂. Reaction was left stirring for 12h under H₂ pressure. Afterwards, reaction was filtered though a celite column and precipitated over cold diethylether. Ether was removed by centrifugation and the gel obtained was dried over vacuum.

Yield: 70%. ¹H-NMR: Doxy-NH-Gly-Gly-Z (300 MHz, MeOD) δ 7.64(m, 1H, C8 Doxy-NH-), 7.26 (5H+1 H C7 Doxy-NH-), 4.96 (s, 2H), 3.69 (d, 2H), 3.57 (2H),etc.
MS analysis: Doxy-NH-Gly-Gly (deprotected): [M+1]=617.20g/mol.

### Synthesis of PGA-CONH-AA-DOXY (Fig. 8.)

PGA (52mg, 0.4mmol unit of glutamic acid) was placed in a round bottom flask and dissolved in anhydrous DMF (5mL) under agitation and N₂ atmosphere. Carboxyl pendant groups were activated with DMTMM·BF₄ (i.e. for 30% activation, 19mg, 0.06mmol), which were added to main reaction previously dissolved in anhydrous DMF. After 10min, AA-doxy-NH₂ was added already dissolved in anhydrous DMF. pH was assessed to be 8. Reaction was left under agitation for 24h protected from light at room temperature. Afterwards, solvent was removed by evaporation and residue was washed with MeOH at 4°C. After remove the supernatant by centrifugation, solid product was dried.
Yield: 50%.

### PURIFICATION OF PGA-X-DOXY CONJUGATES

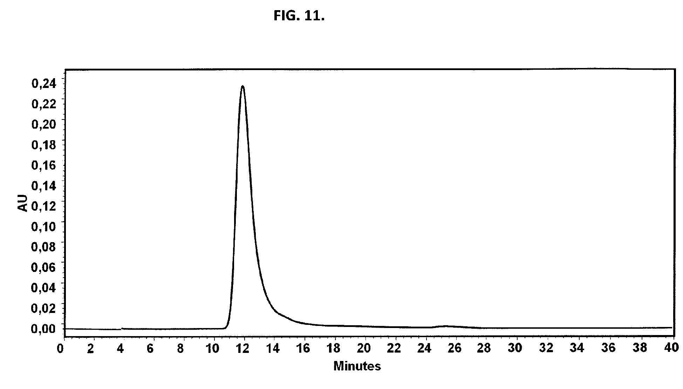

PGA-X-Doxy encompasses all doxycycline conjugates, independently of the type of linkage/spacer between the drug and the polymeric carrier.
After obtaining the polymer conjugate salt form (NaHCO₃ 1M), crude was purified by SEC chromatography (G25 / G10; ddH₂O as eluent). Fractions were analysed by HPLC/GPC techniques (wavelength =273nm). Retention time = 12min. Method: isocratic gradient with PBS 0.1M pH=7.4 as mobile phase, flow rate=1mL/min, 50min. Fractions containing the conjugate were mixed and after lyophilisation, compound was characterised. Example of product chromatogram is shown in Figure 11.

### Example 2. Physico-chemical characterisation of the polymer-drug conjugates.

In this example, it is determined the drug loading, the polymer-drug conjugates stability in different media (plasma, hydrolytical conditions, etc) and their conformation in solution. PGA-X-Doxy encompasses all doxycycline conjugates, independently of the type of linkage/spacer between the drug and the polymeric carrier.

### Determination of total drug loading by UV spectroscopy

For quantifying doxy content, drug weight percentage was determined. Previously, a calibration curve of the parent drug was done. A stock solution of PGA-X-Doxy conjugate in H₂O was prepared (1m/mL). To obtain appropriate and reproducible absorbance measurements, samples were diluted using H₂O. Total drug loading of the conjugates was determined by measuring the optical density at 273nm in H₂O. PGA in the same concentration range as conjugate analysed (0-5mg/mL) in H₂O was used as blank.

### Determination of free drug content by LCMS

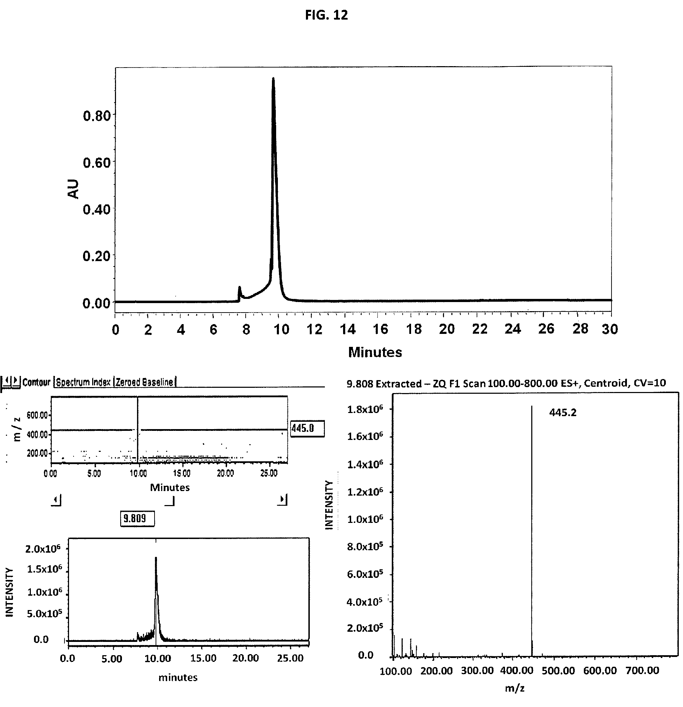

PGA-X-Doxy conjugates were dissolved in phosphate saline buffer (pH=7.4). Aliquots (100uL, 6mg/mL PGA-X-conj) were injected directly in the LC-MS without further extraction (approx. 20uL, Kinetex column (2.6um, C18, 100A 100x4.6mm, mobile phases ddH2O/ACN both 0.1% formic acid). Doxy and doxy-NH2 were able to be detected (Mw=444g/mol, M⁺445g/mol; Mw=459g/mol, M⁺460g/mol, respectively) at retention time around 10minutes (Figure 12). Direct evaluation of PBS aliquots did not detect free doxy (detection limit <0.012mg/mL)hereafter remaining volume was evaporated and solid was washed and sonicated with MeOH. After centrifugation, supernatants were evaluated by LCMS. Again, in all cases drug signals were undetectable concluding that free drug was in the accepted limit for clinical development. In parallel, free drug detection by HPLC was carried out. PGA-X-Doxy conjugates were dissolved in phosphate saline buffer (pH=7.4). Aliquots (100uL, 6mg/mL PGA-X-conj) were lyophilised and residue was washed with MeOH (100uL). Following centrifugation (12000rpm, 5min, 4°C), each supernatant was purified through a POROS 50R2 column to extract the free drug. 1mL fractions were collected and analysed by HPLC (RP-18 column, flow rate =1 mL/min, solvent A: acetonitrile 0.1% trifluoroacetic acid, solvent B: H₂O 0.1%trifluoroacetic acid; method: t= 0 min, B 95%, t= 33 min B 5%, t= 35 min B 5%, t= 40 min B 95%, wavelength =273nm). Retention time of doxy in those conditions is 13.7min.
As a routine, no peaks presence determined a free drug concentration under the limit of detection (0.01 mg/mL) and rarely concentrations were approximately 0.098wt%.

### Drug release studies under hydrolytical conditions

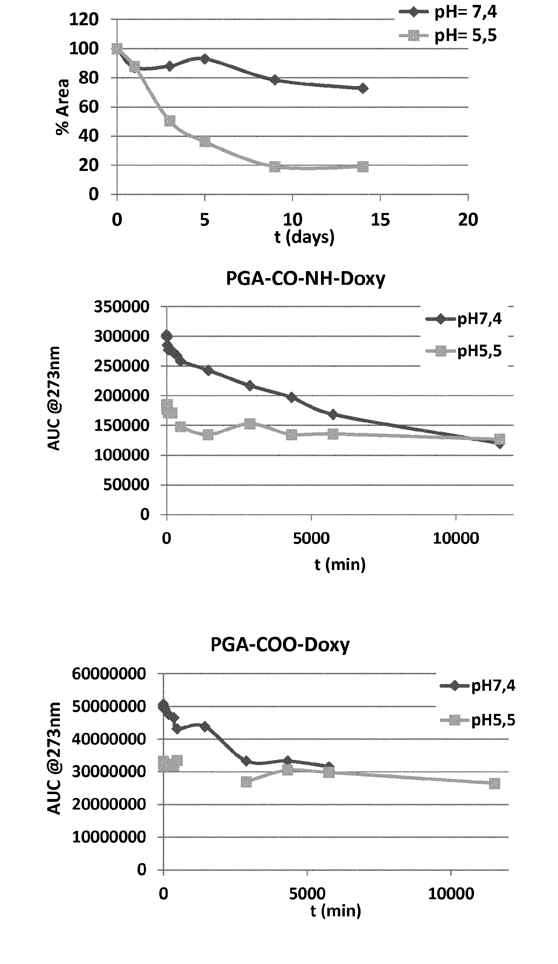

The ability of the conjugates of the present invention to release Doxy in the presence of different pHs was tested. pH 5.5 and 7.4 were evaluated. The results are presented in Figure 13. Conjugates (6 mg/mL) were incubated at 37°C in PBS at pH 5.5 and 7.4 for 17 days. Daily pH was checked and adjusted if necessary. 100µL of the sample solutions were taken at various time. Neutralisation of pH with ammonium phosphate buffer (1 M) before MW lost analysis was performed in order to stop further degradation (Retention time = 12min. Method: isocratic gradient with PBS 0.1 M pH=7.4 as mobile phase, flow rate=1mL/min, 50min). In parallel, free drug of samples at t=0 and 17d was evaluated to quantify drug release (conditions of plasma stability or free drug evaluation studies explained before).
Conjugates with amide bonding/spacer demonstrated stability under both pH conditions and time tested, while conjugates bearing ester bonds showed drug release in a time-and pH-dependent manner.

### Stability of PGA-X-Doxy conjugates in plasma

Stability in plasma was assessed by incubating each of the conjugates (6mg/mL), for 24h at 37°C in plasma freshly extracted from rodents. Free drug was used as a positive control and serum without compound as negative one. At several time points (0min, 1 h, 4h, 8h and 24h), aliquots of 100uL were collected. To each sample, 100uL of ACN was added to precipitate the serum proteins. After centrifugation (12000rpm, 5min, 4°C), supernatants were collected and subjected to analysis by HPLC as described above. Pellets were washed with MeOH (100uL) to extract the free drug out of the pellets. To redissolve the free drug in MeOH, the solid was vortexed and then sonicated. Subsequent to centrifugation (12000 rpm, 5 min, 4°C), supernatant 2 was collected and analyzed by HPLC as reported below. The amount of Doxy release form the conjugates in the presence of serum was determined to be insignificant.

### Stability of the PGA-X-Doxy conjugates in in vitro conditions

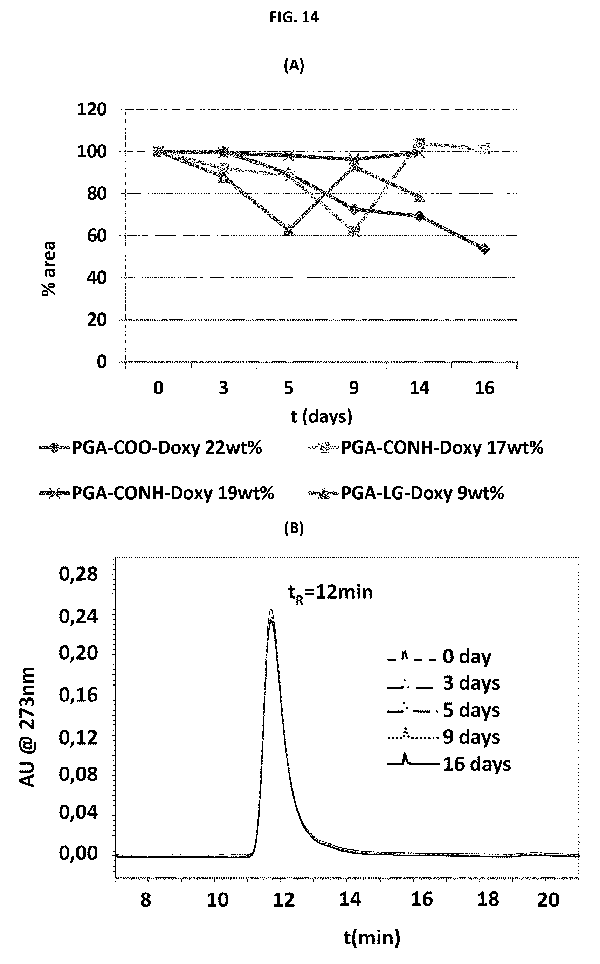

Fibril disruption studies were carried out by incubation of the conjugates with TTR fibrils in PBS media at 37°C. To test if the drug was released from the polymer platform during the in these conditions, a PBS stability-test with the same parameters was set up. Conjugates (6 mg/mL) in PBS 0.1 M were incubated at 37°C at pH=7.4 for 16 days. Sterile conditions were used. 100µL of the sample solutions were taken at different time points up to 16 days. MW loss was evaluated with GPC columns in a HPLC system using methodology and parameters described before. Furthermore, free drug extraction was performed and evaluated with LCMS system. Figure 14 (A/B) shows the results of the study.

### Stability of PGA-X-Doxy conjugates in plasma

Stability in plasma was assessed by incubating each of the conjugates (3-6mg/mL), for 24h at 37°C in plasma freshly extracted from rodents. Free drug was used as a positive control and serum without compound as negative one. At several time points (0min, 1 h, 4h, 8h and 24h), aliquots of 100uL were collected. To each sample, 100uL of ACN was added to precipitate the serum proteins. After centrifugation (12000rpm, 5min, 4°C), supernatants were collected and subjected to analysis by HPLC as described above. Pellets were washed with MeOH (100uL) to extract the free drug out of the pellets. To redissolve the free drug in MeOH, the solid was vortex and then sonicated. Subsequent to centrifugation (12000 rpm, 5 min, 4°C), supernatant 2 was collected and analysed by HPLC as reported below. The amount of Doxy release form the conjugates in the presence of serum was determined to be insignificant.

### Conformation in solution of PGA-CONH-Doxy conjugates through Small Angle Neutron Scattering (SANS) studies.

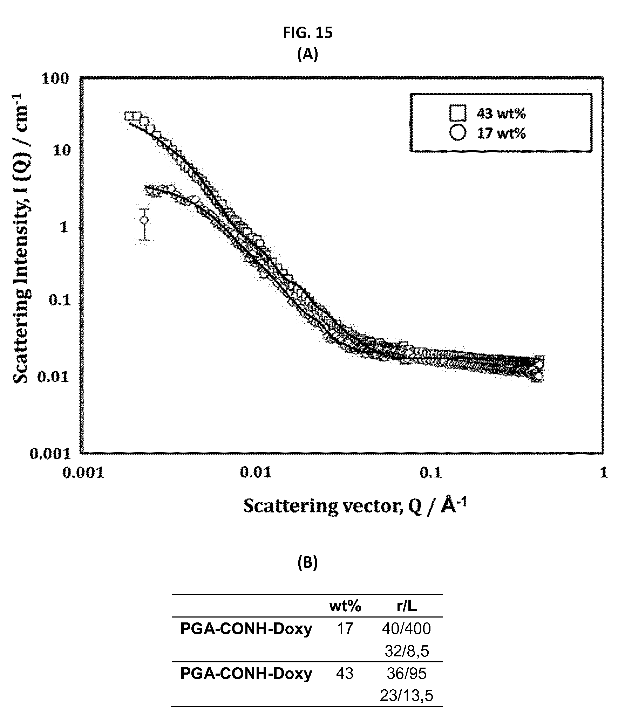

Conformation in solution is a useful tool for explaining conjugates activity either *in vitro* or *in vivo.* Drug availability in the adopted conformation might be directly related with the observed activity. With the aim of elucidate *in vitro* activity differences observed for PGA-doxy conjugates, two compounds with different drug loading, 17 and 43%, were studied by SANS. The samples were evaluated at 0.5-2wt% in a 1 g scale in D2O (pH=5.5, 0.1 M PBS) and were place in quartz cells with an optical path of 2mm, maintaining a the temperature of 37°C (±0.2). The obtained results (Fig. 15A/B) showed a similar conformation of both conjugates that do not fit to simple models such as coils or spheres, but in any case, it could be concluded that even if the conjugate with greater loading (43ωt%) seemed to have a larger hydrodynamic radius, there were not significant changes in the solution conformation when both conjugates were compared.

### Example 3: In vitro activity studies of the polymer-drug conjugates: TTR-fibril

### disruption studies of the PGA-X-Doxy conjugates

### Preparation of amyloid fibrils

TTR Leu55Pro was dialysed against water at pH=7.4 during 24h at 4°C. The solution was centrifuged and the pellet was washed, resuspended in sterile PBS and quantified by Lowry method. Sample concentration was adjusted to 1mg/mL. Protein was later incubated at 37°C for 10-13 days until fibril formation was ratified by Transmission Electron Microscopy (TEM).

### Screening for TTR fibril disrupters

Previous findings revealed that Doxy acts as a TTR fibril disrupter *in vitro* and *in vivo* [4]. All PGA-X-Doxy conjugates synthesised were tested for their ability as disrupters compared with the parent drug (doxy). These includes (1) Doxy-PGA conjugates through ester bond, (2) Doxy-NH₂ derivative PGA conjugates through amide bound and (3) through peptidic linkers, with different drug loadings.
Stock solutions of all compounds were prepared and filtered-sterile prior to use (3.6mM in sterile H₂O). As controls, PGA, Doxy and Doxy-NH₂ were tested. Aliquots of TTR L55P fibrils were prepared and the conjugates and controls were added in a concentration of 6.7-fold molar excess of Doxy (180µM). All aliquots were analysed by TEM and Dynamic Light Scattering (DLS) after 3 and 6 days to monitor fibril disruption.

### Transmission electron microscopy (TEM)

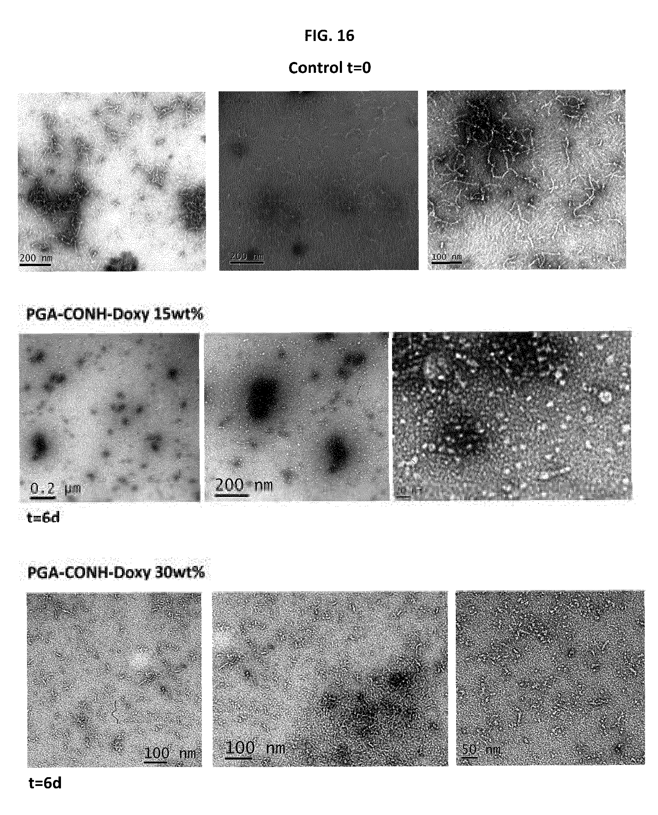

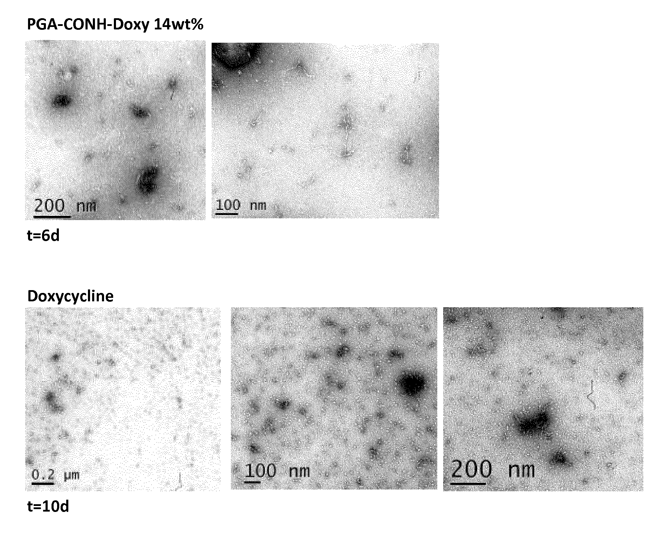

For TEM analysis, sample aliquots were taken under sterile environment, vortexed and adsorbed to glow-discharged carbon-coated collodion film supported on 200-mesh copper grids. For negative staining, the grids were washed with deionized water and stained with 1% uranyl acetate solution. Example of the resulted images are shown in Figure 16.

Visualisation of samples by TEM revealed that, at a concentration of [6.7x10⁻⁵/100µg fibrils] and in presence of the conjugates, fibrils were disrupted after 3 or 6 days due to the appearance of fibrils of smaller extension or a complete disaggregation (Figure 16). Several conjugates demonstrated higher activity as fibril disrupters than parent drug at same drug equivalents.

### Dynamic Light Scattering (DLS)

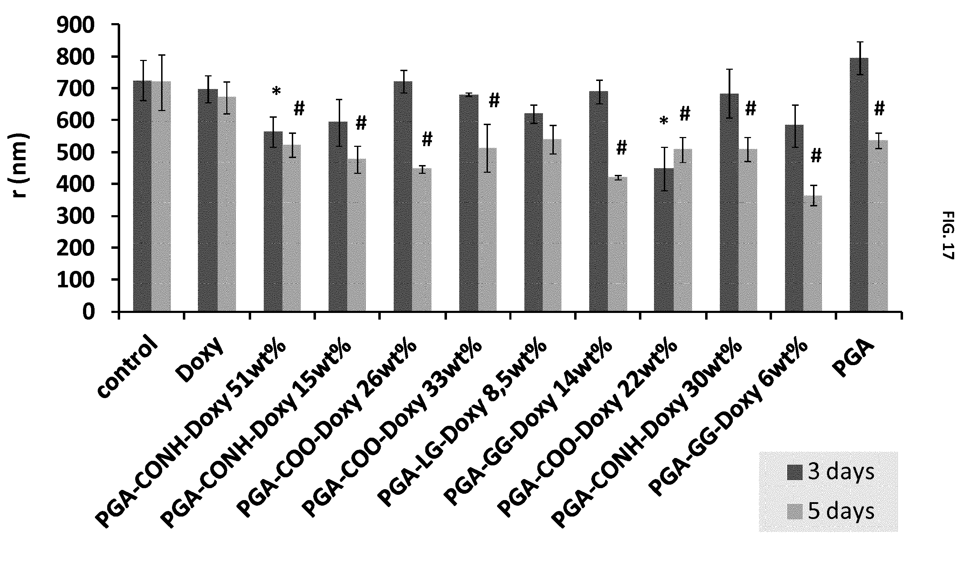

The same samples used in TEM analysis were also analysed by DLS as a complementary technique, in order to observe variations respect to fibrils control after treatment with PGA-X-Doxy conjugates. Results are shown in Figure 17.

### Example 4. Haemolysis assay of the synthesised polymer-drug conjugates

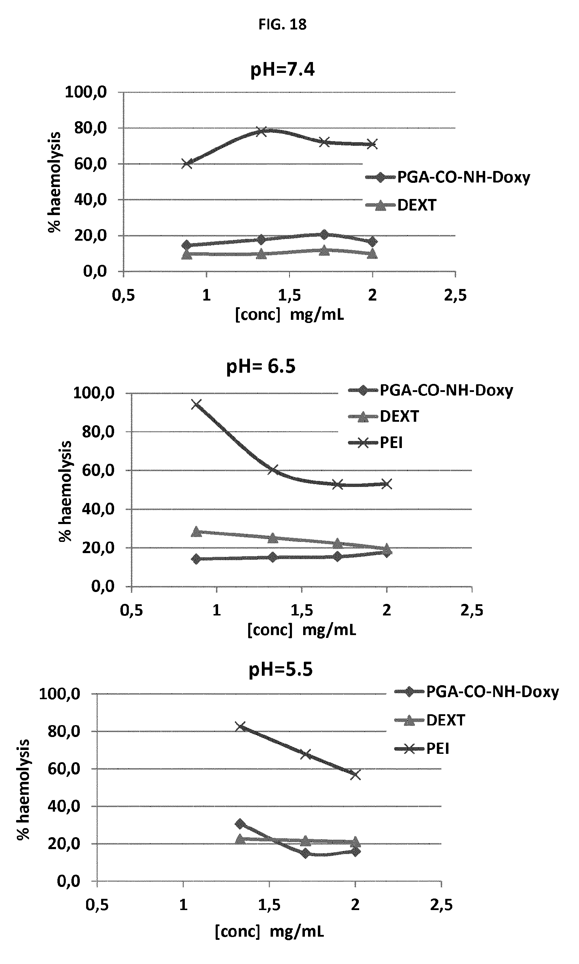

As a proof of suitability of the nanoconjugates for i.v. injection in *in vivo* studies, the haemolytic activity of PGA-Doxy conjugates was tested.
Freshly prepared PGA-X-Doxy, dextran (Mw=74000g/mol) and poly(ethyleneimine) (PEI; Mn~60000, Mw~750000, 50wt% in H₂O) solutions (range of concentrations 0-2mg/mL), in PBS at pH 7.4 and 5.5 were plated (100uL) into sterile 96-well microtitre plates. Blood was taken from adult mice by cardiac puncture immediately after death (by 4% CO₂ asphyxiation) and placed in a heparinised tube on ice. Erythrocytes (RBC) were isolated by centrifugation at 3000rpm for 10min at 4°C. The plasma supernatant was discarded and the RBCs were washed three times using phosphate buffer saline solution. After the third wash, the RBC pellet was equally divided into two and resuspended in PBS solutions with the appropriate pH values (i.e. 7.4, 6.5 and 5.5). From each RBCs solution, it was added (100uL) to the previously prepared microtitre plates containing the test compounds. As 100% reference of lysis, Tritron X-1%w/v was used as well as PBS as reference. Plates were incubated for 16h at 37°C. Then, plates were centrifuged at 3000rpm for 10min at RT. The supernatant were then placed in a new 96-well microtitre plate and haemoglobin (H_{b}) release was measured spectrophotometrically (OD₅₇₀). H_{b} release for each sample was expressed as percentage of the release produced by Triton X. PEI and dextran represented the positive and the negative control, respectively. All haemolysis experiments were carried out per triplicate.
Example of the conjugate PGA-CONH-Doxy 17wt% is shown in Figure 18. As expected, compounds are not haemolytic and therefore appropriate for i.v. administration.

### Example 5: In vivo evaluation studies of the polymer-drug conjugates

### Synthesis of fluorescently labelled conjugates

By means of conjugation of a near infrared dye (Cyane 5.5), conjugates were labelled for posterior biodistribution studies using optical imaging, monitoring *in vivo* as well as *ex vivo* fluorescence.

### PGA-CONH-Doxy conjugate fluorescence labelling

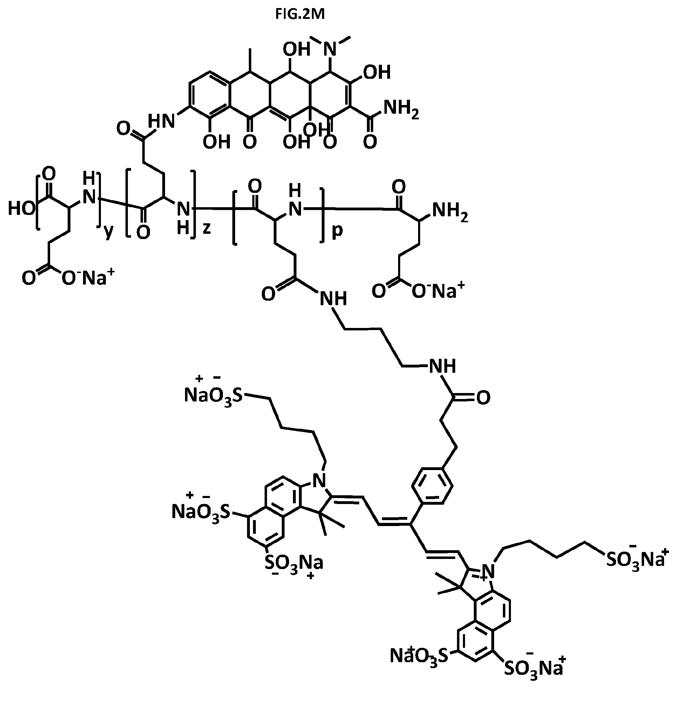

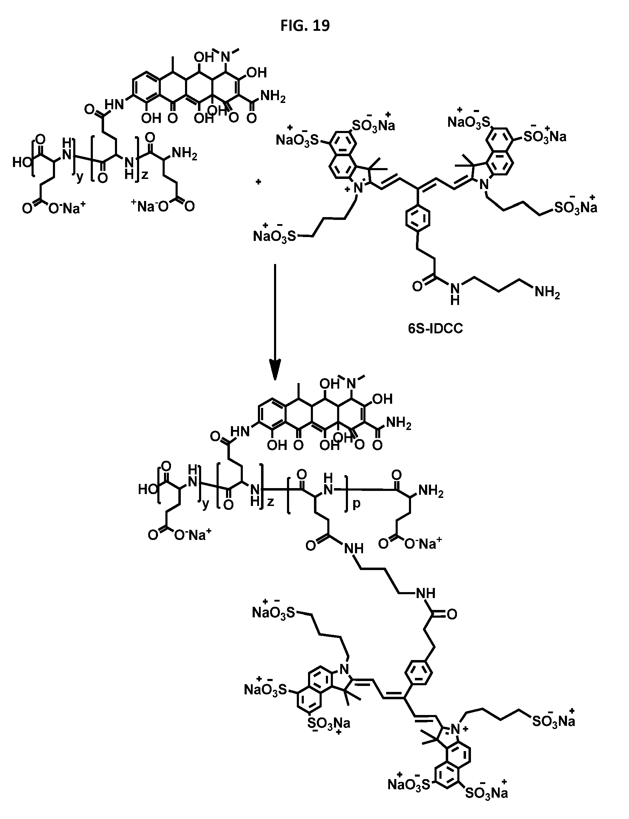

The general derivation procedure of PGA-DOXY fluorescence labelling (Figure 2M.) is depicted in Figure 19.
Briefly, 15mg of PGA-CONH-Doxy conjugate (19ωt% (6.8mol), average MW unit of glutamic acid=180.4/mol) was added to a round bottom flask and dissolved in 3mL of borate buffer pH=9. Then, 1-ethyl-3-(3dimethylaminopropyl)carbodiimide (EDC, 155.24g/mol, 0.0032 mmol) were added and 10min later, N-hydroxysulfosuccinimide (sulfo-NHS, 0.00013mmol, 217.13g/mol) was incorporated. Finally, Cy5.5 (6-SIDCC, 0.011mmol, 1362g/mol) diluted in 1mL of buffer solution was poured into the reaction. Reaction mixture was left under agitation at RT for 24h. After freeze-drying the mixture, residue was purified with a PD-10 column packed with Sephadex™ G-25 (fractions of 500µL) and fluorescence was measured (595/680nm) and fractions with the conjugate were joined. To ensure the absence of free Cy in the conjugate, final product was washed with ddH₂O with Vivaspin® MWCO 3000g/mol.
Yield: 60%. Labelling efficiency: 0.94mol%Cy5.5.

Polymer-drug conjugates biodistribution studies in mice by optical imaging technique
Healthy Balb/c mice (9-12month old) were used as mouse model for the *in vivo* biodistribution studies. For *in vivo* monitoring experiments, animals were first anesthetised and shaved to remove hair interference in fluorescence signal. The *in vivo* whole-body biodistribution as well as *ex vivo* monitoring of the labelled conjugate was performed through noninvasively fluorescence imaging (FLI) using the IVIS®Spectrum Imaging System. Animal care has handled in accordance to guidelines for the Care and Use of Laboratory Animals of the Centro de Investigación Príncipe Felipe, and the experimental procedures were approved by the Animal Experimentation Ethical Committee of the institution.

### PGA-CONH-Doxy-Cy5.5 conjugate biodistribution

Biodistribution of cyane5.5 labelled PGA-CONH-Doxy conjugate (17wt%) (Figure 2M.) was evaluated by optical imaging technique. Conjugate (was dissolved in serum saline

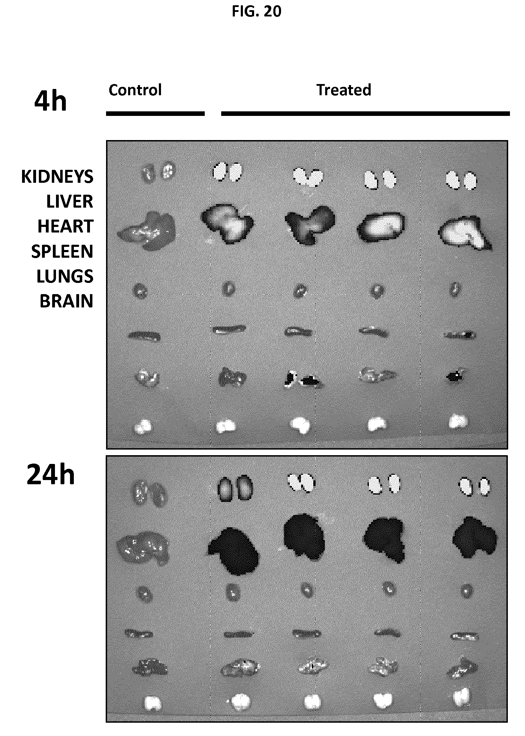

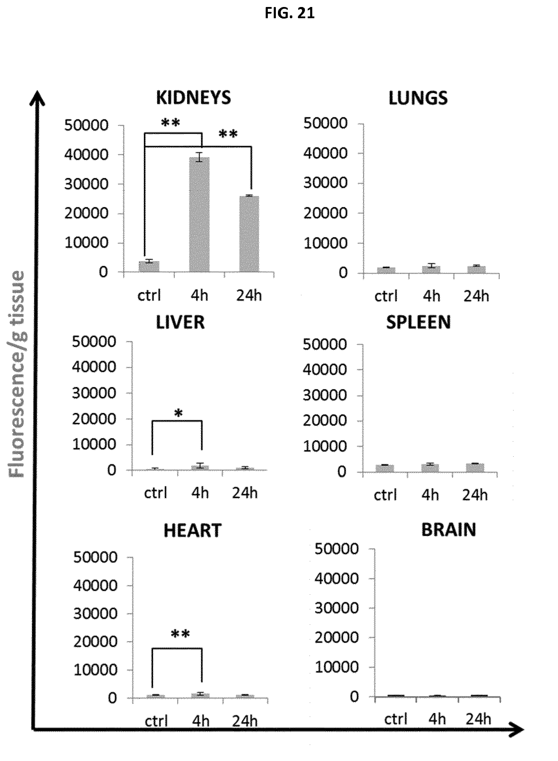

(1.40mg/mL) and animals were injected intravenously a single dose of 100uL of the solution. As controls, non-injected mice were used. Animals were anesthetized using 1-3% isofluorane. Five mice were imaged at a time and imaging settings were set depending on the fluorescence of the animals. The light emitted from the labelled conjugate was detected, digitalised and electronically displayed as a pseudocolor overlay onto a grey scale animal image. Fluorescent signals were quantified as Efficiency. Mice were euthanized at 0, 4 and 24h post administration. Blood was collected and major organs (liver, lungs, spleen, kidneys, heart and brain) were dissected from mice and fluorescence images of excised tissues were obtained by ex *vivo* FLI. Tissues samples were harvested and stored at -80°C to quantify fluorescence. Images of *in vivo* and *ex vivo* monitoring are shown in Fig. 20 and 21.
Organs were homogenised in phosphate buffer solution (PBS pH=7.4) at a specific concentration by means of Ultraturrax device (aprox. 1 min, 13000rpm). Suspension was centrifuged for 1h at 4000g at 4°C and supernatants were collected for fluorescence measurement. Data quantification is shown in Fig. 21. To assess conjugate total extraction, pellets were washed with ddH₂O but no significant signal was observed. For blood samples, immediately after extraction they were centrifuge (10min, 4000rpm, 4°C) and plasma (supernatant) and pellet were frost in liquid nitrogen after analysis. Plasma was directly measured (100uL) per triplicate in 96 well-plate by fluorescence detector. Results showed no specific accumulation in any organ and secretion by kidney and urine.

### Preliminary activity studies in FAP mice model.

All animals were kept and used strictly in accordance with national rules and European Communities Council Directive (86/609/EEC), and all studies performed were approved by the Portuguese General Veterinarian Board (authorization number 024976 from DGV- Portugal). Transgenic mice for human V30M TTR in a TTR null background were kindly provided by Professor Suichiro Maeda from Yamanashi University. In previous studies, these animals were previously analysed and ∼60% of the animals over 1 year of age were found to have TTR deposition as amyloid, i.e., Congo red (CR)-positive material [36] having non-fibrillar TTR deposits at younger ages.
A group of animals (9-11 month of age, n=8) were treated intravenously with the conjugate PGA-CONH-Doxy detailed in Table 1. Animals were given 2 doses per week during 6 weeks. Non-treated mice were used as controls.

Animals were killed after anesthesia with ketamine/xylazine. Organs (including liver, kidney, esophagus, stomach, small and large intestines, heart, spleen, pancreas) were immediately excised and processed. It is important to note that in this model the gastrointestinal track present the majority of fibril accumulation. Tissues were fixed in 4% neutral buffered formalin and embedded in paraffin or immediately frozen, for light microscopy or for total protein extraction, respectively.

**Table 1:**

| **Grupo** | **Conjugado** | **wt%** | **Cantidad de fármaco por dosis** |
|---|---|---|---|
| **A** | PGA-CONH-Doxy | 43 | 8mg drug/Kg |

### Immunohistochemistry (IHC) studies of the organs from the treated mice

TTR-non fibrilar deposition in the extracted tissues was evaluated by immunohistochemistry as well as possible toxicity of these deposits (apoptosis).

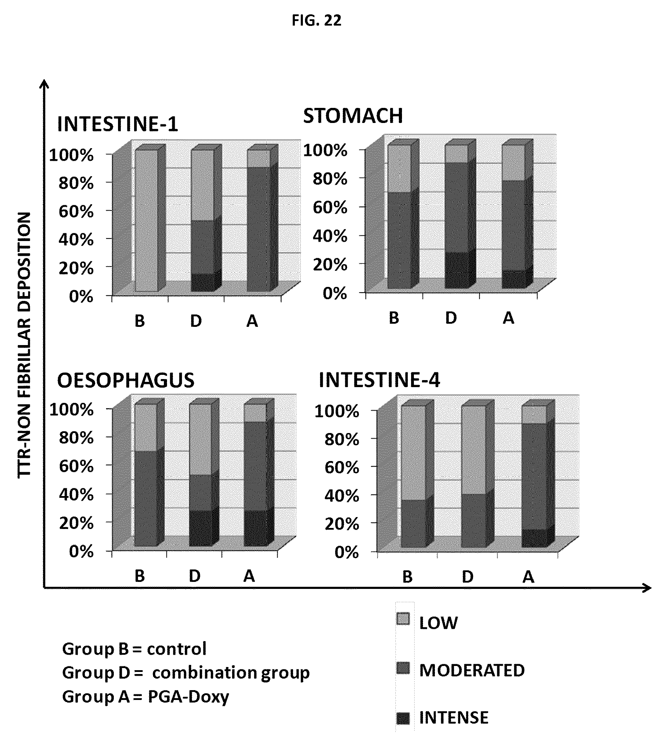

5 µm-thick sections were deparafinated in xylol and dehydrated in a descendent alcohol series. Endogenous peroxidase activity was inhibited with 3% hydrogen peroxide/100% methanol and sections were blocked in 4% bovine serum and 1% bovine serum albumin in phosphate buffered solution (PBS). Primary antibodies used were rabbit polyclonal anti- TTR (Dako, 1:1000), rabbit polyclonal anti-Fas (St Cruz, 1:200), and the rabbit polyclonal anti-Bip (St Cruz, 1:50), which were diluted in blocking solution and incubated overnight at 4°C. Antigen visualization was performed with the biotin-extravidin-peroxidase kit (Sigma Aldrich), using 3-amino-9-ethyl carbazole (Sigma) or diaminobenzidine as substrates. In Fig. 22, there is represented semi-quantitatively the results obtained for non-fibrillar TTR deposition.
In the FAP animal model used, the gastrointestinal (GI) tract is the most affected organ regarding fibrillar deposition. Immunohistochemistry results (Fig. 22) were mainly similar outcomes although
Briefly, Fas plays a key role in apoptotic cell death regulation (induced in this experiment by the non-fibrillar TTR). Fas is a tumour-necrosis receptor that binds the Fas ligand resulting in death-inducing signalling complex ending in the apoptosis cascade. BiP is a molecular chaperon synthesised in higher levels when protein folding is disturbed. Then, the process is directly related to cell stress. When drug stops disease advance, levels of Fas and Bip shoud decrease.
Immunostainning experiments of Fas and BiP did not produce any profitable result. Treated animals had the same pattern than control animals with disease.

### Histological analysis of the organs from the treated mice

For evaluating conjugates toxicity, histological analysis of the tissue sections was performed. This technique enables a better assessment of severe toxicity. Paraffin-embedded sections were stained with hematoxylin/eosin solution (H&E). H&E-stained slice sections were thoroughly examined under low (25 × and 50 ×) and medium (100 × and 200×) magnification. Organs analysed were liver, small and large intestine and liver.

Following examination, staining slices from all groups and organs showed a normal morphology with non toxic evidences due to the treatment. Exception was found for liver of the control group with disease, where focal necrosis, moderate inflammatory infiltrate and vascular congestion was observed. Figure 24 ilustrates some of the results of the experiment.
Summarising, the findings indicate that treated animals did not show toxicity attributed to the administered conjugate. Furthermore, all animals treated showed normal patterns of liver regarding tissue morphology than the disease control animals. Although TTR is not affected by TTR deposits in this organ, liver is the main producer of the protein. Conjugate administered did not provoke toxicity in the analysed organs. The illustrated groups in Fig. 22 are:
Group A: group treated with the conjugate PGA-Doxy.
Group B: control group (animals with disease).
Group C: control group (animals without disease, healthy mice).

### BIBILOGRAPHY

1. M.J. Saraiva, et al., Family studies of the genetic abnormality in transthyretin (prealbumin) in Portuguese patients with familiar amyloidotic polyneuropathy. Ann NY Acad Sci, 1984. 43: p. 86-100.
2. Cardoso, I., et al., Transthyretin fibrillogenesis entails the assembly of monomers: a molecular model for in vitro assembled transthyretin amyloid-like fibrils. J. Mol. Biol., 2002. 317(5): p. 683-695.
3. Cardoso, I., G. Merlini, and M.J. Saraiva, 4'-iodo-4'-deoxydoxorubicin and tetracyclines disrupt transthyretin amyloid fibrils in vitro producing noncytotoxic species: screening for TTR fibril disrupters. FASEB J, 2003. 17(8): p. 803-9.
4. Cardoso, I., et al., Comparative in vitro and ex vivo activities of selected inhibitors of transthyretin aggregation: relevance in drug design. Biochem J, 2007. 408(1): p. 131-8.
5. I. Cardoso and M.J. Saraiva, Doxycycline disrupts transthyretin amyloid: evidence from studies in a FAP transgenic mice model. The FASEB Journal, 2006. 20: p. 234-239.
6. Cardoso, I., et al., Synergy of combined Doxycycline/TUDCA tretment in loweing Transthyretin deposition and associated biomarkers: studies in FAP mouse models. Journal of Translational Medicine, 2010. 8(74).
7. http://clinicaltrials.gov/ct2/show/NCT00439166.
8. Molloy, D.W., et al., A multicenter, blinded, randomized, factorial controlled trial of doxycycline and rifampin for treatment of Alzheimer's disease: the DARAD trial. Int J Geriatr Psychiatry, 2012.
9. Loeb, M.B., et al., A randomized, controlled trial of doxycycline and rifampin for patients with Alzheimer's disease. J Am Geriatr Soc, 2004. 52(3): p. 381-7.
10. Forloni, G., et al., Anti-amyloidogenic activity of tetracyclines: studies in vitro. FEBS Lett, 2001. 487(3): p. 404-7.
11. Familian, A., et al., Inhibitory effect of minocycline on amyloid beta fibril formation and human microglial activation. Glia, 2006. 53(3): p. 233-40.
12. Ryu, J.K., et al., Minocycline inhibits neuronal death and glial activation induced by beta-amyloid peptide in rat hippocampus. Glia, 2004. 48(1): p. 85-90.
13. Monteiro, F.A., et al., In vitro inhibition of transthyretin aggregate-induced cytotoxicity by full and peptide derived forms of the soluble receptor for advanced glycation end products (RAGE). FEBS Lett, 2006. 580(14): p. 3451-6.
14. R. Costa, et al., Testing the therapeutic potential of doxycycline in a Drosophila melanogaster model of Alzheimer disease. J Biol Chem, 2011. 286(48): p. 41647-55.
15. J. Buxbaum, J. Koziol, and L.H. Connors, Serum transthyretin levels in senile systemic amyloidosis: effects of age, gender and ethnicity. Amyloid, 2008. 15(4): p. 255-61.
16. R. Duncan, The dawning era of Polymer Therapeutics. Nature Rev Drug Discov, 2003. 2(5): p. 347.
17. M.J. Vicent, H.R., R. Duncan, Polymer therapeutics: Clinical applications and challenges for development. Adv Drug Deliv Rev., 2009. 61 (13): p. 1117.
18. M. J. Vicent, et al., Polymer therapeutics designed for a combination therapy of hormone-dependent cancer. Angew Chem Int Ed Engl, 2005. 44(26): p. 4061-6.
19. F. Greco and M.J. Vicent, Combination therapy: opportunities and challenges for polymer-drug conjugates as anticancer nanomedicines. Adv Drug Deliv Rev, 2009. 61(13): p. 1203-13.
20. C. Deladriere, R. Lucas, and M.J. Vicent, Future trends, challenges and opportunities with polymer-based combination therapy in cancer. Drug Delivery in Oncology: from basic research to cancer therapy, 2011 (Wiley-VCH Verlag GmbH & Co. KGaA): p. 805-837.
21. M. J. Vicent, H. Ringsdorf, and R. Duncan, Polymer therapeutics: Clinical applications and challenges for development. Adv Drug Deliv Rev., 2009. 61(13): p. 1117.
22. Duncan, R., Polymer therapeutics as nanomedicines: new perspectives. Curr Opin Biotech, 2011. 22.
23. Duncan, R., H. Ringsdorf, and R. Satchi-Fainaro, Polymer therapeutics-polymers as drugs, drug and protein conjugates and gene delivery systems: past, present and future opportunities. J Drug Target, 2006. 14(6): p. 337-41.
24. F. Canal, J. Sanchis, and M.J. Vicent, Polymer-drug conjugates as nano-sized medicines. Curr opin Biotech, 2011. 22(894-900).
25. R. M. England, I. Conejos-Sánchez, and M.J. Vicent, Drug delivery strategies: Polymer Therapeutics. 2012. Chapter 8.2(RSC publishing): p. 456-482.
26. R. Duncan and R. Gaspar, Nanomedicine(s) under the microscope. Mol Pharm, 2011. 8(6): p. 2101-41.
27. J. Sanchis, et al., Polymer-drug conjugates for novel molecular targets. Nanomedicine, 2010. 5(6): p. 915-935.
28. Sabbatini, P., et al., A phase II trial of paclitaxel poliglumex in recurrent or persistent ovarian or primary peritoneal cancer (EOC): a Gynecologic Oncology Group Study. Gynecol Oncol, 2008. 111(3): p. 455-60.
29. Chipman, S.D., et al., Biological and clinical characterization of paclitaxel poliglumex (PPX, CT-2103), a macromolecular polymer-drug conjugate. Int J Nanomedicine, 2006. 1(4): p. 375-83.
30. Lim, W.S., et al., Leukemia-selective uptake and cytotoxicity of CPX-351, a synergistic fixed-ratio cytarabine:daunorubicin formulation, in bone marrow xenografts. Leuk Res, 2010. 34(9): p. 1214-23.
31. Lim, W.S., et al., Schedule- and dose-dependency of CPX-351, a synergistic fixed ratio cytarabine:daunorubicin formulation, in consolidation treatment against human leukemia xenografts. Leuk Lymphoma, 2010. 51(8): p. 1536-42.
32. Harasym, T.O., B.D. Liboiron, and L.D. Mayer, Drug ratio-dependent antagonism: a new category of multidrug resistance and strategies for its circumvention. Methods Mol Biol, 2010. 596: p. 291-323.
33. http://www.celator.ca.
34. Bendeck, M.P., et al., Doxycycline modulates smooth muscle cell growth, migration, and matrix remodeling after arterial injury. Am J Pathol, 2002. 160(3): p. 1089-95.
35. Heenan, R.K., FISH data analysis program. Rutherford Appleton Laboratory, Didcot, U.K.

## Claims

1. A polymer-drug conjugate compound of general formula I, 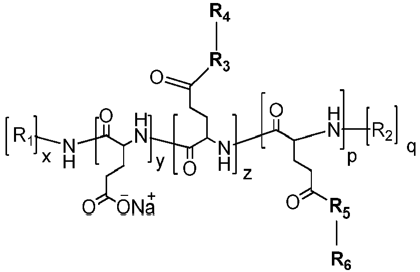 Where the polymeric backbone bears, at least, a bioactive agent, and optionally a second bioactive agent, optionally a probe for the monitoring of the conjugate and optionally a targeting moiety, and
Wherein:
R₁ represents an alkyl group, defined C-terminal linking point (alkyne, azide, thiol, activated thiols, halides, alkenes, activated esters, activated alcohols, protected amines, maleimide group, acetals, activated carboxylic groups), ethylenglycol (EG) of different molecular weights including poly(ethylene glycol) (PEG from 100 to 20000g/mol).
R₂ represents a hydrogen atom, an alkyl group, defined C-terminal linking point (alkyne, azide, thiol, activated thiols, halides, alkenes, activated esters, activated alcohols, protected amines, maleimide group, acetals, activated carboxylic groups), ethylenglycol (EG) of different molecular weights including poly(ethylene glycol) (PEG from 100 to 20000g/mol), PEG-thiol, PEG-4TP.
R₃ represents the linking spacer or bond between the polymer main chain and the bioactive agent (R₄) as itself or derivated, and is an alkyl group, defined C-terminal linking point (alkyne, azide, thiol, activated thiols, halides, alkenes, activated esters, activated alcohols, protected amines, maleimide group, acetals, activated carboxylic groups), ethylenglycol (EG) of different molecular weights including poly(ethylene glycol) (PEG from n=2-16), aminoacids such as lysine, arginine, imidazol, histidine, cysteine and secondary and tertiary amino groups and aminoacid sequences.
R₄ is the selected drug for amyloidosis treatment, selected from the group which comprises the anthracycline antibiotics such as tetracycline, rolitetracycline, minocycline, doxycycline and their derivatives, and the drug can be covalent linked to the polymer chain as itself or previously derivatised (including the introduction of amine, thiol, carbonyl, vinyl, alcohol or carboxyl groups).
R₅ represents the linking spacer or bond between the polymer main chain and the bioactive agent R₆ as itself or derivatised and is an alkyl group, defined C-terminal linking point (alkyne, azide, thiol, activated thiols, halides, alkenes, activated esters,
activated alcohols, protected amines, maleimide group, acetals, activated carboxylic groups), ethylenglycol (EG) of different molecular weights including poly(ethylene glycol) (PEG from n=2 to n=16), aminoacids such as lysine, arginine, imidazol, histidine, cysteine and secondary and tertiary amino groups and aminoacid sequences.
R₆ is the second selected drug for amyloidosis treatment, selected from the group which comprises the anthracycline antibiotics such as tetracycline, rolitetracycline, minocycline, doxycycline and their derivatives (the drug can be covalent linked to the polymer chain as itself or previously derivated); or a labelling moiety exploited for conjugate monitoring, for biodistribution experiments or as a diagnostic probe where the labelling agent comprises fluorescent probes for optical imaging such as Cy5.5, coordination complexes for MRI, or tracers for PET and SPECT, including the chelating agents DTPA, DOTA, NOTA, NODA and metallic ligands such as gallium, technetium, gadolinium, indium.
x is the monomer units included in R₁, from 1 to 1000.
y is an integer having a value such that y/(x+y+z+p+q) multiplied by 100 is in the range of from 0.01 to 99.9
z is an integer having a value such that z/(x+y+z+p+q) multiplied by 100 is in the range of from 0.01 to 99.9
p is an integer having a value such that z/(x+y+z+p+q) multiplied by 100 is in the range of from 0.01 to 99.9
q is the monomer units included in R₂, from 1 to 1000.
R2, R3 and R5 can be used for conjugation of bioactive agents (including low molecular weight drugs, peptides, proteins, antibodies), near infrared dyes, coordination complexes for MRI, PET and SPECT tracers,
and/or their salts, polymorphs, solvates and hydrates for use in the treatment or diagnosis of amyloidosis related diseases.

2. A polymer-drug conjugate for use according to claim 1, wherein:
- There is a conjugated drug in R4, linked to the polymer backbone directly or through a spacer (R3),
- There is a conjugated drug in R4 (linked to the polymer backbone directly or through a spacer (R3)) and a targeting moiety in R6 (linked to the polymer backbone directly or through a spacer) or in R2.
- There is a conjugated drug in R4 (linked to the polymer backbone directly or through a spacer (R3)); a targeting moiety in R6 (linked to the polymer backbone directly or through a spacer) or in R2; and a labelling probe for diagnosis in R6 (linked to the polymer backbone directly or through a spacer) or in R2.
- There is a conjugated drug in R4 (linked to the polymer backbone directly or through a spacer (R3)) and a labelling probe for diagnosis in R6 (linked to the polymer backbone directly or through a spacer) or in R2.
- There are two drugs conjugated in R4 and R6 (linked to the polymer backbone directly or through a spacer (R3 and R5, respectively)).
- There are two drugs conjugated in R4 and R6 (linked to the polymer backbone directly or through a spacer (R3 and R5, respectively)), a targeting moiety in R6 (linked to the polymer backbone directly or through a spacer) or in R2; and a labelling probe for diagnosis in R6 (linked to the polymer backbone directly or through a spacer) or in R2.
- There are two drugs conjugated in R4 and R6 (linked to the polymer backbone directly or through a spacer (R3 and R5, respectively)) and a labelling probe for diagnosis in R6 (linked to the polymer backbone directly or through a spacer) or in R2.
- There are two drugs conjugated in R4 and R6 (linked to the polymer backbone directly or through a spacer (R3 and R5, respectively)) and a targeting moiety in R6 (linked to the polymer backbone directly or through a spacer) or in R2.

3. A polymer-drug conjugate for use according to any of claims 1 and 2, selected from the group consisting of: PGA-COO-Doxycycline, PGA-CONH-Doxycycline, PGA-Leu-Gly-Doxycycline, PGA-Gly-Gly-Doxycycline, PGA-Doxycycline-Cy5.5, PGA-Doxycycline-DOTA/Ga, with different drug/labelling probe loading in any group.

4. A polymer-drug conjugate for use according to any of claims 1- 3 **characterised by** containing a bioactive agent or targeting moiety loading in the polymer higher than 0.5% molar.

5. A polymer-drug conjugate for use according to claims 1 - 4, wherein the treatment or diagnosis of amyloidosis related diseases is selected from the familial amyloidotic polyneuropathy (FAP) and the Alzheimer's Disease (AD).

6. A polymer-drug conjugate for use according to any of claims 1 - 5, together with at least an acceptable pharmaceutical vehicle.

7. A polymer-drug conjugate for use according to claim 6 formulated for its parenteral, oral, topic, nasal and/or rectal administration.

8. A method for obtaining the polymer-drug conjugates of claims 1 to 4, which comprises the next steps:
(a) Co-polymerising a plurality of monomeric units of the polymer, at least one of the monomeric units termination by a first reactive group, and at least one of the monomeric units terminating by a second reactive group, to thereby obtain a copolymer that comprises a plurality of backbone units, at least one backbone unit having the first reactive group and at least one backbone unit having the second reactive group, the first reactive group being capable of reacting with the targeting moiety and the second reactive being capable of reacting with the therapeutically active agent, or:
(b) Polymerisation of a single monomeric unit by means of a polymer block as initiator, and/ or
Post-modification after polymerisation of the first polymeric synthesised block achieving two or more different reactive ending groups in the starting polymer chain with modifiable polymer lengths to the original backbone, giving different final polymeric structures and /or conformations in solution, and/or
Construction of a triblock polymer based structure where the block in between posses the first reactive group being capable of reacting with the therapeutical agent(s) and/or the labelling agent and one of the ending polymeric blocks terminates by a second reactive group being capable of reacting with the labelling agent, the targeting moiety or a second therapeutic agent.
(c) Reacting the polymer carrier with the targeting moiety or a derivative thereof, via the first reactive group, to thereby obtain a polymeric vehicle having the targeting moiety attached to a polymeric backbone thereof; and
(d) Reacting the polymer carrier with the therapeutically active agent or a derivative thereof, via the second reactive group, to thereby obtain the polymer vehicle having the therapeutically active agent attached to a polymeric backbone thereof, thereby obtaining the conjugate of formula I or reacting the polymer carrier with the first therapeutically active agent or a derivative thereof, and secondly the next therapeutically agent (already linked to another polymer or to be linked to the post-modified initial polymer) to the main polymer chain.

## Patentansprüche

1. Polymer-Arzneistoff-Konjugat-Verbindung der allgemeinen Formel I, 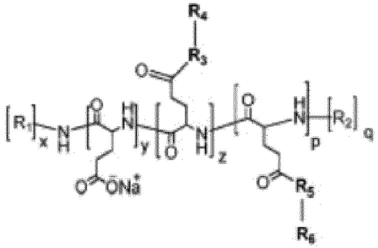 wo das Polymergerüst mindestens ein bioaktives Mittel und wahlweise ein zweites bioaktives Mittel, wahlweise eine Sonde zur Überwachung des Konjugats und wahlweise einen zielgerichteten Anteil trägt, und
wobei:
R₁ eine Alkylgruppe, definierten C-terminalen Verknüpfungspunkt (Alkin, Azid, Thiol, aktivierte Thiole, Halogenide, Alkene, aktivierte Ester, aktivierte Alkohole, geschützte Amine, Maleimid-Gruppe, Acetale, aktivierte Carboxygruppen), Ethylenglykol (EG) verschiedener Molekulargewichte, einschließlich Poly(ethylenglycol) (PEG von 100 bis 20000g/mol), darstellt.
R₂ ein Wasserstoffatom, eine Alkylgruppe, definierten C-terminalen Verknüpfungspunkt (Alkin, Azid, Thiol, aktivierte Thiole, Halogenide, Alkene, aktivierte Ester, aktivierte Alkohole, geschützte Amine, Maleimid-Gruppe, Acetale, aktivierte Carboxygruppen), Ethylenglycol (EG) mit verschiedenen Molekulargewichten, einschließlich Poly(ethylenglycol) (PEG von 100 bis 20000g/mol), PEG-Thiol, PEG-4TP, darstellt.
R₃ den Verknüpfungs-Spacer oder die Bindung zwischen der Polymerhauptkette und dem bioaktiven Mittel (R₄), es selbst oder abgeleitet, darstellt, und eine Alkylgruppe, definierter C-terminaler Verknüpfungspunkt (Alkin, Azid, Thiol, aktivierte Thiole, Halogenide, Alkene, aktivierte Ester, aktivierte Alkohole, geschützte Amine, Maleimid-Gruppe, Acetale, aktivierte Carboxygruppen), Ethylenglycol (EG) mit verschiedenen Molekulargewichten, einschließlich Poly(ethylenglycol) (PEG von n=2-16), Aminosäuren wie Lysin, Arginin, Imidazol, Histidin, Cystein und sekundären und tertiären Aminogruppen und Aminosäuresequenzen, ist.
R₄ der ausgewählte Arzneistoff zur Behandlung der Amyloidose, ausgewählt aus der Gruppe, welche die Anthracyclin-Antibiotika, wie Tetracyclin, Rolitetracyclin, Minocyclin, Doxycyclin und ihre Derivate umfasst, ist, und der Arzneistoff an die Polymerkette, sie selbst oder vorher abgeleitet (einschließlich der Einführung von Amin, Thiol, Carbonyl, Vinyl, Alkohol oder Carboxygruppen), konvalent verknüpft werden kann.
R₅ den Verknüpfungs-Spacer oder die Bindung zwischen der Polymerhauptkette und dem bioaktiven Mittel R₆, es selbst oder abgeleitet, darstellt, und eine Alkylgruppe, definierter C-terminaler Verknüpfungspunkt (Alkin, Azid, Thiol, aktivierte Thiole, Halogenide, Alkene, aktivierte Ester, aktivierte Alkohole, geschützte Amine, Maleimid-Gruppe, Acetale, aktivierte Carboxygruppen), Ethylenglycol (EG) mit verschiedenen Molekulargewichten, einschließlich Poly(ethylenglycol) (PEG von n=2 bis n=16), Aminosäuren, wie Lysin, Arginin, Imidazol, Histidin, Cystein und sekundären und tertiären Aminogruppen und Aminosäuresequenzen, ist.
R₆ der zweite ausgewählte Arzneistoff zur Behandlung der Amyloidose, ausgewählt aus der Gruppe, welche die Anthracyclin-Antibiotika, wie Tetracyclin, Rolitetracyclin, Minocyclin, Doxycyclin und ihre Derivate (der Arzneistoff kann an die Polymerkette, sie selbst oder vorher abgeleitet, kovalent verknüpft werden) umfasst; oder ein Markierungsanteil, der zur Konjugat-Überwachung, für Experimente der biologischen Verteilung oder als diagnostische Sonde genutzt wird, wo das Markierungsmittel Fluoreszenzsonden zur optischen Bildgebung, wie Cy5.5, Koordinationskomplexe für MRI, oder Tracer für PET und SPECT, einschließlich der Quelating-Mittel DTPA, DOTA, NOTA, NODA und Metallliganden wie Gallium, Technetium, Gadolinium, Indium umfasst, ist.
x die Monomereinheiten, enthalten in in R₁, von 1 bis 1000, ist.
y eine ganze Zahl mit einem Wert, dass y/(x+y+z+p+q) multipliziert mit 100 im Bereich von 0.01 bis 99.9 liegt, ist.
z eine ganze Zahl mit einem Wert, dass z/(x+y+z+p+q) multipliziert mit 100 im Bereich von 0.01 bis 99.9 liegt, ist.
p eine ganze Zahl mit einem Wert, dass z/(x+y+z+p+q) multipliziert mit 100 in einem Bereich von 0.01 bis 99.9 liegt, ist.
q die Monomereinheiten, enthalten in R₂, von 1 bis 1000, ist.
R2, R3 und R5 können zur Konjugation von bioaktiven Mitteln (einschließlich niedermolekularen Arzneistoffen, Peptiden, Proteinen, Antikörpern), in der Nähe von Infrarot-Farbstoffen, Koordinatenkomplexen für MRI, PET und SPECT-Tracern,
und/oder ihre Salze, Polymorphe, Solvate und Hydrate zum Gebrauch bei der Behandlung oder Diagnose von Krankheiten im Zusammenhang mit Amyloidose, verwendet werden.

2. Polymer-Arzneistoff-Konjugat zum Gebrauch nach Anspruch 1, wobei:
- ein konjugierter Arzneistoff in R4, verknüpft mit dem Polymergerüst, entweder direkt oder über einen Spacer (R3), vorhanden ist,
- ein konjugierter Arzneistoff in R4 (verknüpft mit dem Polymergerüst, entweder direkt oder über einen Spacer (R3)), und ein zielgerichteter Anteil in R6 (verknüpft mit dem Polymergerüst, entweder direkt oder über einen Spacer) oder in R2, vorhanden ist.
- Ein konjugierter Arzneistoff in R4 (verknüpft mit dem Polymergrüst, entweder direkt oder über einen Spacer (R3)); ein zielgerichteter Anteil in R6 (verknüpft mit dem Polymergerüst entweder direkt oder über einen Spacer) oder in R2; und eine Markierungssonde für die Diagnose in R6 (verknüpft mit dem Polymergerüst, entweder direkt oder über einen Spacer) oder in R2, vorhanden ist.
- Ein konjugierter Arzneistoff in R4 (verknüpft mit dem Polymergerüst, entweder direkt oder über einen Spacer (R3)), und eine Markierungssonde für die Diagnose in R6 (verknüpft mit dem Polymergerüst, entweder direkt oder über einen Spacer) oder in R2, vorhanden ist.
- Zwei Arzneistoffe, konjugiert in R4 und R6 (verknüpft mit dem Polymergerüst, entweder direkt oder über einen Spacer (R3 und R5, entsprechend)), vorhanden sind.
- Zwei Arzneistoffe, konjugiert in R4 und R6 (verknüpft mit dem Polymergerüst, entweder direkt oder über einen Spacer (R3 und R5, entsprechend)), ein zielgerichteter Anteil in R6 (verknüpft mit dem Polymergerüst, entweder direkt oder über einen Spacer) oder in R2; und eine Markierungssonde für die Diagnose in R6 (verknüpft mit dem Polymergerüst, entweder direkt oder über einen Spacer) oder in R2, vorhanden sind.
- Zwei Arzneistoffe, konjugiert in R4 und R6 (verknüpft mit dem Polymergerüst, entweder direkt oder über einen Spacer (R3 und R5, entsprechend)) und eine Markierungssonde für die Diagnose in R6 (verknüpft mit dem Polymergerüst, entweder direkt oder über einen Spacer) oder in R2, vorhanden sind.
- Zwei Arzneistoffe, konjugiert in R4 und R6 (verknüpft mit dem Polymergerüst, entweder direkt oder über einen Spacer (R3 und R5, entsprechend)) und ein zielgerichteter Anteil in R6 (verknüpft mit dem Polymergerüst, entweder direkt oder über einen Spacer) oder in R2, vorhanden sind.

3. Polymer-Arzneistoff-Konjugat zum Gebrauch nach den Ansprüchen 1 und 2, ausgewählt aus der Gruppe, bestehend aus PGA-COO-Doxycyclin, PGA-CONH-Doxycyclin, PGA-Leu-Gly-Doxycyclin, PGA-Gly-Gly-Doxycyclin, PGA-Doxycyclin-Cy5.5, PGA-Doxycyclin-DOTA/Ga, mit verschiedener Arzneistoff/Markierungssonde in jeder Gruppe.

4. Polymer-Arzneistoff-Konjugat zum Gebrauch nach einem der Ansprüche 1 - 3, **dadurch gekennzeichnet, dass** es ein bioaktives Mittel oder einen zielgerichteten Anteil, mit einer Beladung in dem Polymer von höher als 0,5 % molar, enthält.

5. Polymer-Arzneistoff-Konjugat zum Gebrauch nach den Ansprüchen 1 - 4, wobei die Behandlung oder Diagnose von Krankheiten im Zusammenhang mit Amyloidose aus der familiären Amyloidose Polyneuropathie (FAP) und der Alzheimer-Krankheit (AD) ausgewählt wird.

6. Polymer-Arzneistoff-Konjugat zum Gebrauch nach den Ansprüchen 1 - 5, zusammen mit mindestens einem akzeptablen pharmazeutischen Bindemittel.

7. Polymer-Arzneistoff-Konjugat zum Gebrauch nach Anspruch 6, das für seine parenterale, orale, topische, nasale und/oder rektale Verabreichung formuliert ist.

8. Verfahren zum Erhalten der Polymer-Arzneistoff-Konjugate nach einem der Ansprüche 1 bis 4, das die nächsten Schritte umfasst:
(a) Copolymerisation einer Mehrzahl von Monomereinheiten des Polymers, mindestens eine der Monomereinheiten, die durch eine erste reaktive Gruppe terminiert, und mindestens eine der Monomereinheiten, die durch eine zweite reaktive Gruppe terminiert, um dadurch ein Copolymer zu erhalten, das eine Vielzahl von Gerüsteinheiten umfasst, wobei mindestens eine Gerüsteinheit die erste reaktive Gruppe hat und mindestens eine Gerüsteinheit die zweite reaktive Gruppe hat, wobei die erste reaktive Gruppe imstande ist, mit dem zielgerichteten Anteil zu reagieren und die zweite reaktive Gruppe imstande ist, mit dem therapeutischen Wirkstoff zu reagieren, oder:
(b) Polymerisation einer einzelnen Monomereinheit mittels eines Polymerblocks als Initiator und/oder
Postmodifikation nach der Polymerisation des ersten Polymersynthesierten Blocks, wobei zwei oder mehr verschiedene reaktive Endgruppen in der beginnenden Polymerkette mit modifizierbaren Polymerlängen zum Ausgangsgerüst erzielt werden, was verschiedene endgültige polymere Strukturen und/oder gelöste Konformationen bietet, und/oder
Konstruktion einer auf Triblockpolymer basierenden Struktur, wo der Block dazwischen die erste reaktive Gruppe, die imstande ist, mit dem(n) therapeutischen Mittel(n) und/oder dem Markierungsmittel zu reagieren, besitzt, und eines der endenden Polymerblöcke durch eine zweite reaktive Gruppe, die imstande ist, mit dem Markierungsmittel, dem zielgerichteten Anteil oder dem zweiten therapeutischen Mittel zu reagieren, abschließt.
(c) Reaktion des Polymerträgers mit dem zielgerichtetem Anteil oder einem Derivat davon, über die erste reaktive Gruppe, um dadurch ein PolymerBindemittel zu erhalten, wobei der zielgerichtete Anteil an ein Polymergerüst davon angebunden ist; und
(d) Reaktion des Polymerträgers mit dem therapeutischen Wirkstoff oder einem Derivat davon, über die zweite reaktive Gruppe, um dadurch das PolymerBindemittel zu erhalten, wobei der therapeutische Wirkstoff an ein Polymergerüst davon angebunden ist, wodurch das Konjugat der Formel I erhalten wird oder der Polymerträger mit dem ersten therapeutischen Wirkstoff oder einem Derivat davon reagiert, und zweitens das nächstfolgende therapeutische Mittel (das bereits an ein anderes Polymer gebunden ist oder an das postmodifizierte anfängliche Polymer gebunden wird) an die Haupt-Polymerkette.

## Revendications

1. Composé de conjugué de médicament-polymère de formule générale I, 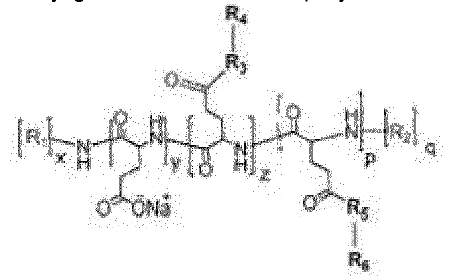 où le squelette polymérique comporte, au moins, un agent bioactif, et optionnellement un deuxième agent bioactif, optionnellement une sonde pour la surveillance du conjugué et optionnellement un fragment de ciblage, et
Dans lequel :
R₁ représente
un groupe alkyle, un point de liaison C-terminal défini (alcyne, azoture, thiol, thiols activés, halogénures, alcènes, esters activés, alcools activés, amines protégés, groupe maléimide, acétals, groupes carboxyliques activés), l'éthylène glycol (EG) de poids moléculaires différents y compris le poly(éthylène glycol) (PEG de 100 à 20000 g/mol).
R₂ représente un atome d'hydrogène, un groupe alkyle, un point de liaison C-terminal défini (alcyne, azoture, thiols, thiols activés, halogénures, alcènes, esters activés, alcools activés, amines protégés, groupe maléimide, acétals, groupes carboxyliques activés), l'éthylène glycol (EG) de poids moléculaires différents y compris le poly(éthylène glycol) (PEG de 100 à 20000 g/mol), le PEG-thiol, le PEG-4TP.
R₃ représente l'espaceur de liaison ou le lien entre la chaîne principale polymère et l'agent bioactif (R₄) lui-même ou dérivé, et est un groupe alkyle, un point de liaison C-terminal défini (alcyne, azoture, thiol, thiols activés, halogénures, alcènes, esters activés, alcools activés, amines protégés, groupe maléimide, acétals, groupes carboxyliques activés), l'éthylène glycol (EG) de poids moléculaires différents y compris le poly(éthylène glycol) (PEG de n=2-16), des aminoacides tels que la lysine, l'arginine, l'imidazole, l'histidine, la cystéine et des groupes amino secondaires et tertiaires et des séquences aminoacides et des séquences aminoacides.
R₄ est le médicament choisi pour le traitement de l'amyloïdose, choisi dans le groupe qui comprend les antibiotiques anthracyclines tels que la tétracycline, la rolitétracycline, la minocycline, la doxycycline et leurs dérivés, et le médicament peut être covalent lié à la chaîne de polymère elle-même ou préalablement dérivatisée (y compris l'introduction d'amine, de thiol, de carbonyle, de vinyle, d'alcool ou de groupes carboxyles)
R₅ représente l'espaceur de liaison ou lien entre la chaîne principale polymère et l'agent bioactif R₆ lui-même ou dérivatisé et est un groupe alkyle, un point de liaison C-terminal défini (alcyne, azoture, thiol, thiols activés, halogénures, alcènes, esters activés, alcools activés, amines protégés, groupe maléimide, acétals, groupes carboxyliques activés), l'éthylène glycol (EG) de poids moléculaires différents y compris le poly(éthylène glycol) (PEG de n=2 à n=16), des aminoacides tels que la lysine, l'arginine, l'imidazole, l'histidine, la cystéine et des groupes amino secondaires et tertiaires et des séquences aminoacides.
R₆ est le deuxième médicament choisi pour le traitement de l'amyloïdose, choisi dans le groupe qui comprend les antibiotiques anthracyclines tels que la tétracycline, la rolitétracycline, la minocycline, la doxycycline et leurs dérivés (le médicament peut être covalent lié à la chaîne de polymère elle-même ou préalablement dérivée) ; ou un fragment d'étiquetage exploité pour la surveillance du conjugué, pour des expériences de biodistribution ou comme sonde diagnostic où l'agent d'étiquetage comprend des sondes fluorescentes pour l'imagerie optique telles que Cy5.5, des complexes de coordination pour l'IRM, ou des traceurs pour la TEP et la TEMP, y compris les agents de chélation DTPA, DOTA, NOTA, NODA et des liants métalliques tels que le gallium, le technétium, le gadolinium, l'indium.
x est l'unité monomère comprise dans R₁, de 1 à 1000.
y est un entier ayant une valeur telle que y/(x+y+z+p+q) multiplié par 100 est dans la plage de 0,01 à 99,9.
z est un entier ayant une valeur telle que z/(x+y+z+p+q) multiplié par 100 est dans la plage de 0,01 à 99,9.
p est un entier ayant une valeur telle que z/(x+y+z+p+q) multiplié par 100 est dans la plage de 0,01 à 99.9.
q est l'unité monomère comprise dans R₂, de 1 à 1000.
R2, R3 et R5 peuvent être utilisés pour la conjugaison d'agents bioactifs (y compris les médicaments de faible poids moléculaire, les peptides, les protéines, les anticorps), de colorants dans le proche infrarouge, de complexes de coordination pour l'IRM, la TEP et les traceurs TEMP,
et/ou leurs sels, polymorphes, solvates et hydrates pour une utilisation dans le traitement ou le diagnostic des maladies associées à l'amyloïdose.

2. Conjugué de médicament-polymère pour une utilisation selon la revendication 1, dans lequel :
- Il y a un médicament conjugué dans R4, lié au squelette de polymère directement ou par le biais d'un espaceur (R3),
- Il y a un médicament conjugué dans R4 (lié au squelette de polymère directement ou par le biais d'un espaceur (R3)) et un fragment de ciblage dans R6 (lié au squelette de polymère directement ou par le biais d'un espaceur) ou dans R2.
- -Il y a un médicament conjugué dans R4 (lié au squelette de polymère directement ou par le biais d'un espaceur (R3)) ; un fragment de ciblage dans R6 (lié au squelette de polymère directement ou par le biais d'un espaceur) ou dans R2 ; et une sonde d'étiquetage pour le diagnostic dans R6 (lié au squelette de polymère directement ou par le biais d'un espaceur) ou dans R2.
- -Il y a un médicament conjugué dans R4 (lié au squelette de polymère directement ou par le biais d'un espaceur (R3)) et une sonde d'étiquetage pour le diagnostic dans R6 (lié au squelette de polymère directement ou par le biais d'un espaceur) ou dans R2.
- Il y a deux médicaments conjugués dans R4 et R6 (liés au squelette de polymère directement ou par le biais d'un espaceur (R3 et R5, respectivement)).
- Il y a deux médicaments conjugués dans R4 et R6 (liés au squelette de polymère directement ou par le biais d'un espaceur (R3 et R5, respectivement)), un fragment de ciblage dans R6 (lié au squelette de polymère directement ou par le biais d'un espaceur) ou dans R2 ; et une sonde d'étiquetage pour le diagnostic dans R6 (lié au squelette polymère directement ou par le biais d'un espaceur) ou dans R2.
- Il y a deux médicaments conjugués dans R4 et R6 (liés au squelette de polymère directement ou par le biais d'un espaceur (R3 et R5, respectivement) et une sonde d'étiquetage pour le diagnostic dans R6 (lié au squelette de polymère directement ou par le biais d'un espaceur) ou dans R2.
- Il y a deux médicaments conjugués dans R4 et R6 (liés au squelette de polymère directement ou par le biais d'un espaceur (R3 et R5, respectivement)) et un fragment de ciblage dans R6 (lié au squelette de polymère directement ou par le biais d'un espaceur) ou dans R2.

3. Conjugué de médicament-polymère pour une utilisation selon l'une quelconque des revendications 1 et 2, choisi dans le groupe consistant en : PGA-COO-Doxycycline, PGA- CONH-Doxycycline, PGA-Leu Gly-Doxycycline, PGA-Gly-Gly-Doxycycline, PGA- Doxycycline-Cy5.5, PGA-Doxycycline-DOTA/Ga, avec une concentration différente de sonde de médicament/étiquetage dans tous les groupes.

4. Conjugué de médicament-polymère pour une utilisation selon l'une quelconque des revendications 1 - 3 **caractérisé en ce qu'**il contient une concentration d'agent bioactif ou de fragment de ciblage supérieure à 0,5 % molaire.

5. Conjugué de médicament-polymère pour une utilisation selon les revendications 1 - 4, dans lequel le traitement ou le diagnostic de maladies associées à l'amyloïdose est choisi parmi la polyneuropathie amyloïde familiale (PAF) et la maladie d'Alzheimer (MA).

6. Conjugué de médicament-polymère pour une utilisation selon l'une quelconque des revendications 1 - 5, conjointement à au moins un véhicule pharmaceutiquement acceptable.

7. Conjugué de médicament-polymère pour une utilisation selon la revendication 6 formulé pour son administration parentérale, orale, topique, nasale et/ou rectale.

8. Procédé pour obtenir les conjugués de médicament-polymère selon les revendications 1 à 4, qui comprend les étapes suivantes :
(a) co-polymériser une pluralité d'unités monomériques du polymère, au moins l'une des unités monomériques terminant par un premier groupe réactif, et au moins l'une des unités monomériques terminant par un deuxième groupe réactif, pour obtenir ainsi un copolymère qui comprend une pluralité d'unités de squelette, au moins une unité de squelette ayant le premier groupe réactif et au moins une unité de squelette ayant le deuxième groupe réactif, le premier groupe réactif étant capable de réagir avec le fragment de ciblage et le deuxième réactif étant capable de réagir avec l'agent actif thérapeutiquement, ou :
(b) Polymérisation d'une seule unité monomérique au moyen d'un polymère bloc comme initiateur, et/ou
Post-modification après la polymérisation du premier bloc synthétisé polymérique obtenant deux ou plusieurs groupes de fin réactifs dans la chaîne de polymère de début avec des longueurs de polymère modifiables au squelette original, donnant différentes structures polymériques finales et/ou conformations dans la solution, et/ou
Construction d'une structure à base de polymère tribloc où le bloc au milieu possède le premier groupe réactif étant capable de réagir avec l'agent (les agents) thérapeutique(s) et/ou l'agent d'étiquetage et l'un des blocs polymériques de fin termine par un deuxième groupe réactif étant capable de réagir avec l'agent d'étiquetage, le fragment de ciblage ou un deuxième agent thérapeutique.
(c) Faire réagir le véhicule polymère avec le fragment de ciblage ou un dérivé de celui-ci, à travers le premier groupe réactif, pour obtenir ainsi un véhicule polymérique ayant le fragment de ciblage attaché à un squelette polymérique de celui-ci ; et
(d) Faire réagir le véhicule polymère avec l'agent actif thérapeutique ou un dérivé de celui-ci, à travers le deuxième groupe réactif, pour obtenir ainsi le véhicule polymère ayant l'agent actif thérapeutique attaché à un squelette polymérique de celui-ci, obtenant ainsi le conjugué de formule 1 ou faisant réagir le véhicule de polymère avec le premier agent réactif thérapeutiquement ou un dérivé de celui-ci, et deuxièmement le prochain agent thérapeutiquement (déjà lié à un autre polymère ou destiné à être lié au polymère initial post-modifié) à la chaîne de polymère principale.
